# EUROPEAN PATENT SPECIFICATION

(11) **EP 2 160 607 B1**
(45) Date of publication and mention of the grant of the patent: **07.09.2011**
(21) Application number: 08756407.6
(22) Date of filing: 29.05.2008
(51) Int. Cl.: G01N 33/58

(54) **ASSAY METHODS FOR IDENTIFYING AGENTS THAT MODIFY THE ACTIVITY OF NAPE-PLD OR ABH4**
TESTVERFAHREN ZUR ERKENNUNG VON WIRKSTOFFEN ZUR MODIFIZIERUNG DER AKTIVITÄT VON NAPE-PLD ODER ABH4
PROCÉDÉS D'ANALYSE POUR IDENTIFIER DES AGENTS QUI MODIFIENT L'ACTIVITÉ DE NAPE-FLD OU ABH4

(30) Priority: 31.05.2007 US 941045 P
(43) Date of publication of application: 10.03.2010
(73) Proprietor: SANOFI, 75013 Paris (FR)
(72) Inventor: PEPPARD, Jane V., Bridgewater, New Jersey 08807 (US); MEHDI, Shujaath, Bridgewater, New Jersey 08807 (US); LI, Zhuyin, Bridgewater, New Jersey 08807 (US); DUGUID, Mei S., Bridgewater, New Jersey 08807 (US)
(74) Representative: Körner, Kathrin
(86) International application number: PCT/US2008/065028
(87) International publication number: WO 2008/150832

(56) References cited:
- HENDRICKSON H STEWART ET AL: "Intramolecularly quenched BODIPY-labeled phospholipid analogs in phospholipase A2 and platelet-activating factor acetylhydrolase assays and in vivo fluorescence imaging" ANALYTICAL BIOCHEMISTRY, ACADEMIC PRESS INC. NEW YORK, vol. 276, no. 1, 1 December 1999 (1999-12-01), pages 27-35, XP002186412 ISSN: 0003-2697 cited in the application

### SEQUENCE LISTING

<110> sanofi-aventis U.S. Inc.
   Peppard, Jane V.
   Mehdi, Shujaath
   Li, Zhuyin
   Duguid, Mei S.
<120> ASSAY METHODS FOR IDENTIFYING AGENTS THAT MODIFY THE ACTIVITY OF
   NAPE-PLD OR ABH4
<130> US2006/230 WO PCT
<160> 5
<170> PatentIn version 3.3
<210> 1
   <211> 1200
   <212> DNA
   <213> Homo sapiens
<400> 1
   atggatgaaa atgaaagcaa ccagtctctg atgacaagca gccaatatcc taaagaagca      60
   gtaagaaaac gtcaaaattc agcacggaat tccggagcaa gtgattcttc taggttttct     120
   aggaaaagct tcaaactgga ttatagacta gaagaagatg taactaaatc caagaaagga     180
   aaagatggga gatttgtgaa tccgtggcca acatggaaaa acccctctat tccaaatgtt     240
   ctcagatggc tgataatgga gaaagatcac agcagtgttc caagttctaa agaggaacta     300
   gacaaagaac tcccagtgct taagccatat tttatcacta accctgaaga agctggagtg     360
   agggaagctg gcttaagagt cacatggctg ggacatgcca cggtaatggt ggaaatggat     420
   gagctcatat ttctcacgga tcccatcttt agctctcgtg cttcaccatc gcagtacatg     480
   ggtccaaagc gatttcgtcg ttccccgtgc acaataagtg aactccctcc aatagatgcg     540
   gtccttatca gtcacaacca ctatgaccat ctggactaca attctgtcat tgctttgaat     600
   gagcgatttg gtaatgagtt gagatggttt gtgcctttgg gtctccttga ctggatgcaa     660
   aaatgtggct gtgagaatgt gattgagttg gactggtggg aggagaattg tgtccccgga     720
   catgataagg tcacttttgt ctttacacct tcccagcact ggtgtaaaag gactctaatg     780
   gatgacaaca aggtgctatg gggcagctgg tctgtcttgg ggccttggaa tcgatttttt     840
   ttcgcaggag atactggtta ttgccctgct tttgaagaga taggaaaaag atttggacct     900
   tttgaccttg cagctattcc catcggagct tatgaaccga ggtggtttat gaaataccag     960
   catgtagacc cagaagaagc tgtaaggatt cacactgatg tccaaacaaa gaaatctatg    1020
   gcaattcact ggggaacttt tgccttagca aatgagcatt acttagagcc tccagtgaag    1080
   ctgaatgaag ctctagagag atacggactt aacgctgaag atttttttgt cttgaagcat    1140
   ggagaatcaa gatacctaaa taatgatgat gaaaactttc atcaccatca ccatcactaa    1200
<210> 2
   <211> 399
   <212> PRT
   <213> Homo sapiens
<400> 2
   Met Asp Glu Asn Glu Ser Asn Gln Ser Leu Met Thr Ser Ser Gln Tyr
   1               5                   10                  15
   Pro Lys Glu Ala Val Arg Lys Arg Gln Asn Ser Ala Arg Asn Ser Gly
               20                  25                  30
   Ala Ser Asp Ser Ser Arg Phe Ser Arg Lys Ser Phe Lys Leu Asp Tyr
           35                  40                  45
   Arg Leu Glu Glu Asp Val Thr Lys Ser Lys Lys Gly Lys Asp Gly Arg
       50                  55                  60
   Phe Val Asn Pro Trp Pro Thr Trp Lys Asn Pro Ser Ile Pro Asn Val
   65                  70                  75                  80
   Leu Arg Trp Leu Ile Met Glu Lys Asp His Ser Ser Val Pro Ser Ser
                   85                  90                  95
   Lys Glu Glu Leu Asp Lys Glu Leu Pro Val Leu Lys Pro Tyr Phe Ile
               100                 105                 110
   Thr Asn Pro Glu Glu Ala Gly Val Arg Glu Ala Gly Leu Arg Val Thr
           115                 120                 125
   Trp Leu Gly His Ala Thr Val Met Val Glu Met Asp Glu Leu Ile Phe
       130                 135                 140
   Leu Thr Asp Pro Ile Phe Ser Ser Arg Ala Ser Pro Ser Gln Tyr Met
   145                 150                 155                 160
   Gly Pro Lys Arg Phe Arg Arg Ser Pro Cys Thr Ile Ser Glu Leu Pro
                   165                 170                 175
   Pro Ile Asp Ala Val Leu Ile Ser His Asn His Tyr Asp His Leu Asp
               180                 185                 190
   Tyr Asn Ser Val Ile Ala Leu Asn Glu Arg Phe Gly Asn Glu Leu Arg
           195                 200                 205
   Trp Phe Val Pro Leu Gly Leu Leu Asp Trp Met Gln Lys Cys Gly Cys
       210                 215                 220
   Glu Asn Val Ile Glu Leu Asp Trp Trp Glu Glu Asn Cys Val Pro Gly
   225                 230                 235                 240
   His Asp Lys Val Thr Phe Val Phe Thr Pro Ser Gln His Trp Cys Lys
                   245                 250                 255
   Arg Thr Leu Met Asp Asp Asn Lys Val Leu Trp Gly Ser Trp Ser Val
               260                 265                 270
   Leu Gly Pro Trp Asn Arg Phe Phe Phe Ala Gly Asp Thr Gly Tyr Cys
           275                 280                 285
   Pro Ala Phe Glu Glu Ile Gly Lys Arg Phe Gly Pro Phe Asp Leu Ala
       290                 295                 300
   Ala Ile Pro Ile Gly Ala Tyr Glu Pro Arg Trp Phe Met Lys Tyr Gln
   305                 310                 315                 320
   His Val Asp Pro Glu Glu Ala Val Arg Ile His Thr Asp Val Gln Thr
                   325                 330                 335
   Lys Lys Ser Met Ala Ile His Trp Gly Thr Phe Ala Leu Ala Asn Glu
               340                 345                 350
   His Tyr Leu Glu Pro Pro Val Lys Leu Asn Glu Ala Leu Glu Arg Tyr
           355                 360                 365
   Gly Leu Asn Ala Glu Asp Phe Phe Val Leu Lys His Gly Glu Ser Arg
       370                 375                 380
   Tyr Leu Asn Asn Asp Asp Glu Asn Phe His His His His His His
   385                 390                 395
<210> 3
   <211> 353
   <212> PRT
   <213> Homo sapiens
<400> 3
   Asp Tyr Arg Leu Glu Glu Asp Val Thr Lys Ser Lys Lys Gly Lys Asp
   1               5                   10                  15
   Gly Arg Phe Val Asn Pro Trp Pro Thr Trp Lys Asn Pro Ser Ile Pro
   20 25 30
   Asn Val Leu Arg Trp Leu Ile Met Glu Lys Asp His Ser Ser Val Pro
   35 40 45
   Ser Ser Lys Glu Glu Leu Asp Lys Glu Leu Pro Val Leu Lys Pro Tyr
   50 55 60
   Phe Ile Thr Asn Pro Glu Glu Ala Gly Val Arg Glu Ala Gly Leu Arg
   65 70 75 80
   Val Thr Trp Leu Gly His Ala Thr Val Met Val Glu Met Asp Glu Leu
   85 90 95
   Ile Phe Leu Thr Asp Pro Ile Phe Ser Ser Arg Ala Ser Pro Ser Gln
   100 105 110
   Tyr Met Gly Pro Lys Arg Phe Arg Arg Ser Pro Cys Thr Ile Ser Glu
   115 120 125
   Leu Pro Pro Ile Asp Ala Val Leu Ile Ser His Asn His Tyr Asp His
   130 135 140
   Leu Asp Tyr Asn Ser Val Ile Ala Leu Asn Glu Arg Phe Gly Asn Glu
   145 150 155 160
   Leu Arg Trp Phe Val Pro Leu Gly Leu Leu Asp Trp Met Gln Lys Cys
   165 170 175
   Gly Cys Glu Asn Val Ile Glu Leu Asp Trp Trp Glu Glu Asn Cys Val
   180 185 190
   Pro Gly His Asp Lys Val Thr Phe Val Phe Thr Pro Ser Gln His Trp
   195 200 205
   Cys Lys Arg Thr Leu Met Asp Asp Asn Lys Val Leu Trp Gly Ser Trp
   210 215 220
   Ser Val Leu Gly Pro Trp Asn Arg Phe Phe Phe Ala Gly Asp Thr Gly
   225 230 235 240
   Tyr Cys Pro Ala Phe Glu Glu Ile Gly Lys Arg Phe Gly Pro Phe Asp
   245 250 255
   Leu Ala Ala Ile Pro Ile Gly Ala Tyr Glu Pro Arg Trp Phe Met Lys
   260 265 270
   Tyr Gln His Val Asp Pro Glu Glu Ala Val Arg Ile His Thr Asp Val
           275                 280                 285
   Gln Thr Lys Lys Ser Met Ala Ile His Trp Gly Thr Phe Ala Leu Ala
       290                 295                 300
   Asn Glu His Tyr Leu Glu Pro Pro Val Lys Leu Asn Glu Ala Leu Glu
   305                 310                 315                 320
   Arg Tyr Gly Leu Asn Ala Glu Asp Phe Phe Val Leu Lys His Gly Glu
                   325                 330                 335
   Ser Arg Tyr Leu Asn Asn Asp Asp Glu Asn Phe His His His His His
               340                 345                 350
   His
<210> 4
   <211> 6130
   <212> DNA
   <213> Artificial
<220>
   <223> pcDNA3.1zeo_DEST vector containing human Abh4
<210> 5
   <211> 348
   <212> PRT
   <213> Homo sapiens
<400> 5
   Met Ala Asp Asp Leu Glu Gln Gln Ser Gln Gly Trp Leu Ser Ser Trp
   1               5                   10                  15
   Leu Pro Thr Trp Arg Pro Thr Ser Met Ser Gln Leu Lys Asn Val Glu
               20                  25                  30
   Ala Arg Ile Leu Gln Cys Leu Gln Asn Lys Phe Leu Ala Arg Tyr Val
           35                  40                  45
   Ser Leu Pro Asn Gln Asn Lys Ile Trp Thr Val Thr Val Ser Pro Glu
       50                  55                  60
   Gln Asn Asp Arg Thr Pro Leu Val Met Val His Gly Phe Gly Gly Gly
   65                  70                  75                  80
   Val Gly Leu Trp Ile Leu Asn Met Asp Ser Leu Ser Ala Arg Arg Thr
                   85                  90                  95
   Leu His Thr Phe Asp Leu Leu Gly Phe Gly Arg Ser Ser Arg Pro Ala
               100                 105                 110
   Phe Pro Arg Asp Pro Glu Gly Ala Glu Asp Glu Phe Val Thr Ser Ile
           115                 120                 125
   Glu Thr Trp Arg Glu Thr Met Gly Ile Pro Ser Met Ile Leu Leu Gly
       130                 135                 140
   His Ser Leu Gly Gly Phe Leu Ala Thr Ser Tyr Ser Ile Lys Tyr Pro
   145                 150                 155                 160
   Asp Arg Val Lys His Leu Ile Leu Val Asp Pro Trp Gly Phe Pro Leu
                   165                 170                 175
   Arg Pro Thr Asn Pro Ser Glu Ile Arg Ala Pro Pro Ala Trp Val Lys
               180                 185                 190
   Ala Val Ala Ser Val Leu Gly Arg Ser Asn Pro Leu Ala Val Leu Arg
           195                 200                 205
   Val Ala Gly Pro Trp Gly Pro Gly Leu Val Gln Arg Phe Arg Pro Asp
       210                 215                 220
   Phe Lys Arg Lys Phe Ala Asp Phe Phe Glu Asp Asp Thr Ile Ser Glu
   225                 230                 235                 240
   Tyr Ile Tyr His Cys Asn Ala Gln Asn Pro Ser Gly Glu Thr Ala Phe
                   245                 250                 255
   Lys Ala Met Met Glu Ser Phe Gly Trp Ala Arg Arg Pro Met Leu Glu
               260                 265                 270
   Arg Ile His Leu Ile Arg Lys Asp Val Pro Ile Thr Met Ile Tyr Gly
           275                 280                 285
   Ser Asp Thr Trp Ile Asp Thr Ser Thr Gly Lys Lys Val Lys Met Gln
       290                 295                 300
   Arg Pro Asp Ser Tyr Val Arg Asp Met Glu Ile Lys Gly Ala Ser His
   305                 310                 315                 320
   His Val Tyr Ala Asp Gln Pro His Ile Phe Asn Ala Val Val Glu Glu
                   325                 330                 335
   Ile Cys Asp Ser Val Asp His His His His His His
               340                 345

## Description

### BACKGROUND OF THE INVENTION

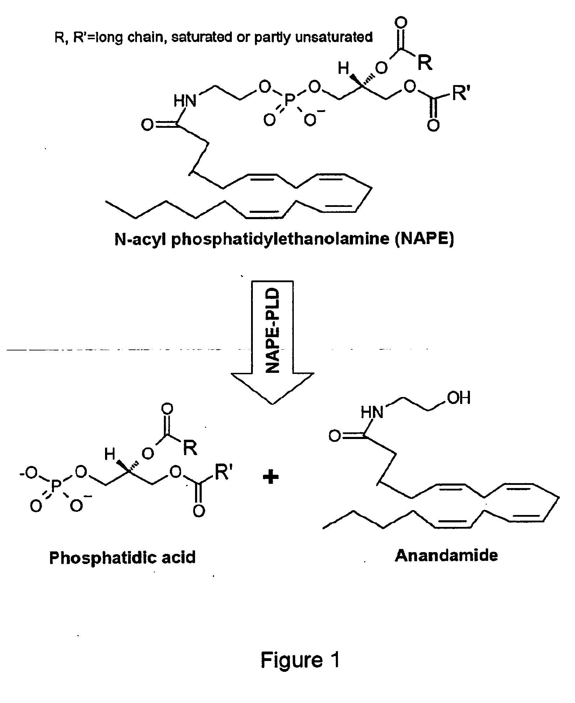

N-acylphosphatidylethanolamine-hydrolysing phospholipase D (NAPE-PLD) enzyme is a phospholipase which catalyzes the hydrolysis of N-acylphosphatidylethanolamine (NAPE) to phosphatidic acid and anandamide (Figure 1). Alpha/beta-hydrolase-4 (Abh4; also abbreviated as Abhd4) produces anandamide by an alternative pathway. Anandamide is one of the known endocannabinoids. Thus, NAPE-PLD and Abh4 are biologically attractive targets, since inhibition of the biosynthesis of anandamide may have some of the same effects as cannabinoid-1 (CB1) antagonists or additional effects. Therefore, therapeutics that modulate the activity of NAPE-PLD or Abh4 may be useful in the treatment of varied disorders such as, but not limited to, obesity, diabetes, and atherosclerosis.

Because of the therapeutic potential of discovering compounds that modulate NAPE-PLD, there was a need in the pharmaceutical industry to develop a high-throughput screening (HTS) assay. However, NAPE-PLD is a unique enzyme. It is structurally and catalytically distinguishable from other lipases including other phospholipase D (PLD) enzymes. Unlike other PLDs which catalyze analogous reactions and are histidine-dependent enzymes, NAPE-PLD is a zinc dependent enzyme, resembling bacterial zinc metallo-beta-lactamases. The N-acyl moiety in the substrate is required for NAPE-PLD substrate recognition while other PLDs use phosphatidylcholine or phosphatidylinositol as substrates (Schmid et al. J. Biol. Chem. 258:9302-9306, 1983; Okamoto et al., J. Biol. Chem. 279:5298-5305, 2004; Gottlin et al., Proc. Natl. Acad. USA 95:9202-9207, 1998; Petersen and Hansen, FEBS Lett. 455:41-44, 1999; Ueda et al., Curr. Med. Chem. 12:1413-1422, 2005). Thus, many of the assay methods for PLDs reported in the literature are not amendable for measuring NAPE-PLD. A spectrophotometric assay measuring p-nitrophenol release from PLD does not involve the N-acyl moiety necessary for NAPE-PLD enzymatic activity (Hagishita et al., Anal. Biochem. 276:161-165, 1999; D'Arrigo et al., Analytica Chimica Acta 304:249-254, 1995). Assays that detect the formation of choline which is the product of many normal PLDs are not useful because NAPE-PLD does not produce choline (Hojjati and Jiang, J. Lipid Res. 47:673-676, 2006). A continuous fluorescence displacement assay for PLD that detects the formation of free fatty acid requires the additional coupling of a phospholipase A enzyme (*i.e*., an indirect measurement of PLD activity) and employs a substrate lacking the N-acyl moiety (Kinkaid and Wilton, Biochem. Soc. Trans. 25:S595, 1997). Other methodologies utilize techniques such as chromotography, pH changes and membrane charge that are not easily detectable in HTS format (Petersen et al., J. Lipid Res. 41:1532-1538, 2000; Morris et al., Anal. Biochem. 252:1, 1997; Aridor et al., Methods Enzymol. 404:108-115, 2005) Several HTS assays for lipases recently developed do not have the required N-acyl moiety (Schmidt and Bomscheuer, Biomol. Eng. 22:51-56, 2005). Unlike NAPE-PLD, Abh4 is not a zinc dependent enzyme but is a serine hydrolase (Simon and Cravatt, J. Biol. Chem. 281:26465, 2006).

Methods reported for assaying NAPE-PLD activity use radiolabeled NAPE followed by quantifying radiolabeled product released by thin layer chromatography (Okamoto et al, J. Biol. Chem. 279:5298-5304, 2004), zirconium precipitation (Petersen et al., J. Lipid Res. 41:1532-1538, 2000), or liquid chromatography (Fezza et al, Analytical Biochemistry 339 :113-120, 2005).

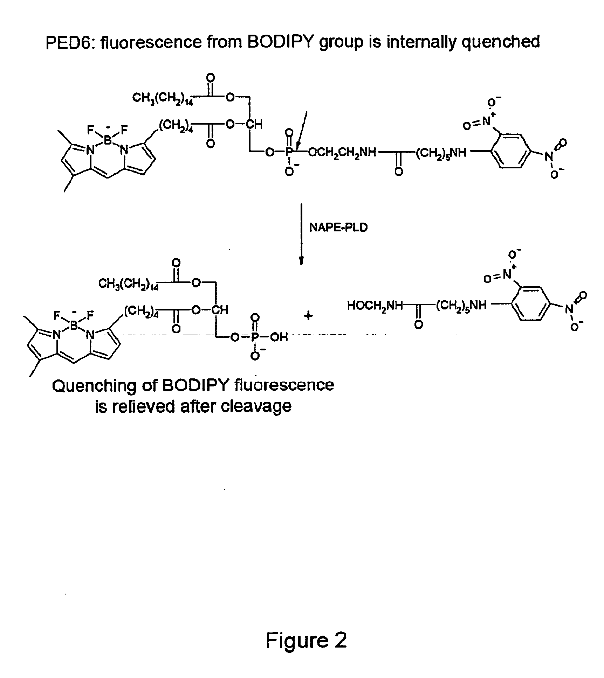

Fluorescent quenching and fluorescent energy transfer methods are commonly employed in HTS formatted assays. However, fluorescent quenching and fluorescent energy transfer methods or any other HTS-enabling methods have not been applied to NAPE-PLD. We have utilized a fluorescent BODIPY group and a quenching 2, 4-dinitrophenyl (DNP) group (Hendrickson et al., Anal. Biochem. 276:27-35, 1999) to develop a novel assay for NAPE-PLD enabling high throughput screening. The HTS compatible NAPE-PLD fluorescence assay is based on a BODIPY and DNP dual labeled N-acylphosphatidylethanolamine molecule (PED6) as the substrate. As an intact molecule, the fluorescence from the BODIPY moiety is quenched by the DNP moiety; upon enzymatic hydrolysis of PED6, the DNP moiety is released from the BODIPY moiety, allowing fluorescence emission from BODIPY (see Figure 2). The fluorescence intensity is proportional to the activity of NAPE-PLD. This new assay increases throughput of the screening, enabling the testing of more compounds in a short time period. Also, this HTS-formatted assay obviates the handling, waste management and disposal issues associated with the use of radioactivity and thin layer chromatography of radiolabeled material.

Abh4 is a member of a family of hydrolases that contain the signature alpha/beta protein fold. Abh4 has been proposed to participate in a pathway for the biosynthesis of the endocannabinoid arachidonlethanolamide (AEA; anandamide) (Simon and Cravatt, J. Biol. Chem. 281:26465, 2006). This pathway has been proposed as an alternative to the pathway that uses the enzyme NAPE-PLD. The assay described by Simon and Cravatt for measuring the enzymatic activity of Abh4 uses a radioactive NAPE substrate, followed by separation by thin layer chromatography of products. Such an assay is cumbersome and clearly not suited to screening compounds with any reasonable throughput. In addition, the use of radioactivity is undesirable for routine assays due to precautionary handling and disposal procedures. The radioactive substrate used by Simon and Cravatt is a NAPE that has no chemical tags, fluorescent or otherwise.

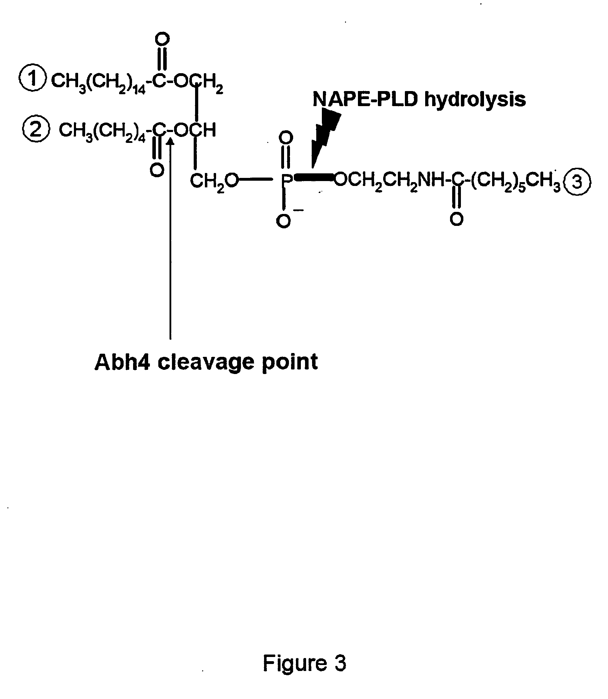

We hypothesized that the NAPE analog PED6, used for the first time successfully by us for measuring NAPE-PLD activity, could be useful for Abh4 as well. We anticipated that Abh4 would cleave the ester bond at the 2-position which is a different bond from that cleaved by NAPE-PLD (see Figure 3), but the end result of an increase in fluorescence upon relief of quenching would be the same. The ester bond at the 1- position may be cleaved as well by Abh4 but will not produce a measurable fluorescence change. The use of the tagged substrate PED6 for the Abh4 enzyme is novel as it is not at all apparent that the enzyme would tolerate the tags in the substrate and hydrolyze PED6 to a measurable extent.

### BRIEF DESCRIPTION OF THE DRAWINGS

Figure 1 shows the hydrolysis of N-acylphosphatidylethanolamine (NAPE) to phosphatidic acid and anandamide by the enzyme N-acylphosphatidylethanolamine-hydrolysing phospholipase D (NAPE-PLD).
Figure 2 shows a schematic representation of an intact molecule of PED6 comprising the fluorescence group of the BODIPY moiety and the quenching group of the DNP moiety. Upon enzymatic hydrolysis by NAPE-PLD, the DNP moiety is released from the BODIPY moiety, allowing fluorescence emission from BODIPY.
Figure 3 shows possible positions of donor and acceptor (or quencher) moieties of any two suitably situated fluorescent molecules, for instance, BODIPY and DNP. Cleavage sites of NAPE-PLD and Abh4 are noted.
Figure 4 shows the effects Triton X-100 and lipids used to prepare PED6 substrate on the signal-to-background ratio of BODIPY fluorescence measuring NAPE-PLD activity. Final Triton X-100 concentration was 0.0125 %. Chloroform was used to dissolve the substrate.
Figure 5 shows the effects of Triton X-100 when ethyl acetate is used to dissolve the PED6 substrate on the signal-to-background ratio of BODIPY fluorescence measuring NAPE-PLD activity.
Figure 6 shows a PED6 substrate titration curve (CPS=counts per second).
Figure 7 shows a NADE-PLD enzyme titration curve (CPS=counts per second).
Figure 8 shows the Km determination for NADE-PLD.
Figure 9 shows a DMSO sensitivity curve for the NADE-PLD assay.
Figure 10 shows representative IC₅₀ curves.

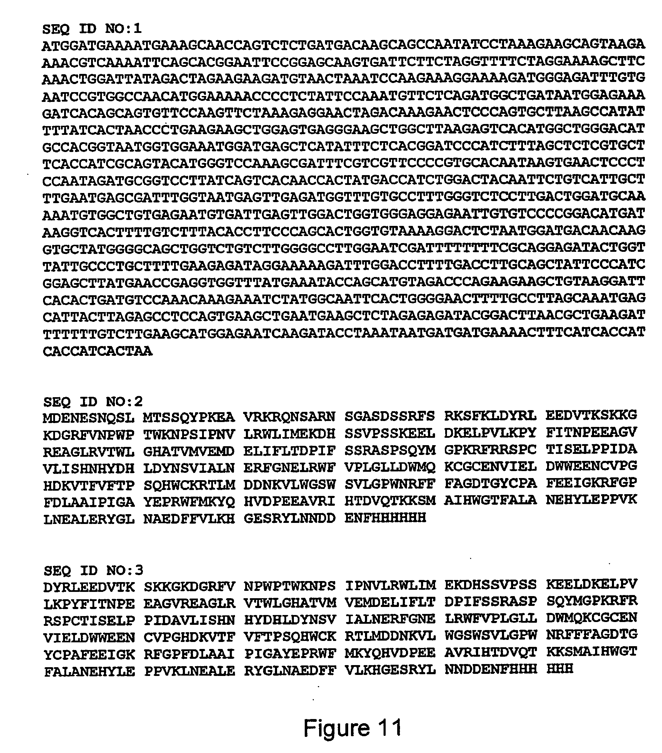

Figure 11 shows the nucleotide sequence (SEQ ID NO:1) used to express NAPE-PLD with a C-terminal 6x His tag; the NAPE-PLD protein with C-terminal His tag that was expressed and purified was found to be truncated at the N-terminus (SEQ ID NO. 3) compared to the sequence of full-length NAPE-PLD (SEQ ID NO. 2).
Figure 12 shows the expressed amino acid sequence of Abh4 with C-terminal His tag (SEQ ID NO: 5).

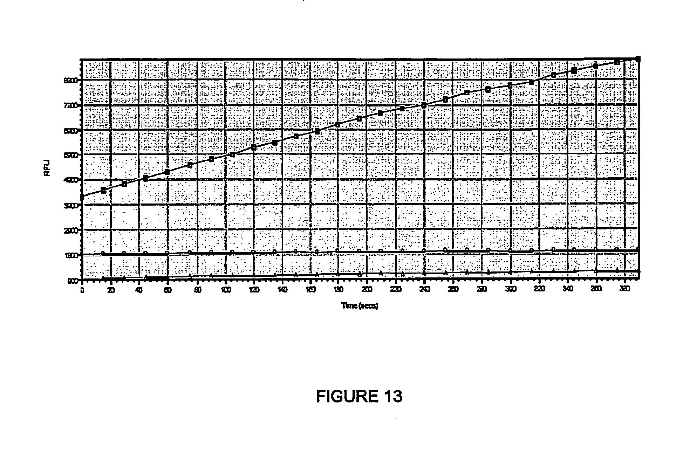

Figure 13 shows representative traces following the fluorescence emission resulting from hydrolysis of PED6 by Abh4 over time (emission wavelength 520nm). Time is in seconds (secs), RFU is relative flourescent units in milli-flourescent units per minute (mRFU/min). A1 (open circle) represents substrate control alone (slope = 17335 mRFU/min), A2 (open square) represents lysate from cells transfected with vector containing Abh4 (slope=849272 mRFU/min), A3 (open triangle) represents lysate from untransfected cells (slope = 41622 mRFU/min).

### DETAILED DESCRIPTION OF THE INVENTION

Unless defined otherwise, all technical and scientific terms used herein have the same meaning as commonly understood to one of ordinary skill in the art to which this invention pertains.

The terminology used in this specification and the appended claims is for the purpose of describing particular embodiments only and use in the specification is not intended to be limiting of the invention. The singular forms of a word are intended to include the plural forms unless the context clearly indicates otherwise. For example, the singular forms of "a", "an" and "the" are intended to include the plural forms as well. Further, reference to an agent may include a mixture of two or more agents. Thus, the term "an agent" includes a plurality of agents, including mixtures and/or enantiomers thereof. It should also be noted that the term "or" is generally employed in its sense including "and/or" unless the context clearly dictates otherwise. It will be further understood that the terms "comprises" and/or "comprising," when used in this specification, specify the presence of stated features, steps, elements, and/or components, but do not preclude the presence or addition of one or more other features, steps, elements, components, and/or groups thereof.

Furthermore, in accordance with the present invention there may be employed conventional molecular biology, microbiology, and recombinant DNA techniques within the skill of the art. Such techniques are explained fully in the literature. See, e.g., Sambrook, Fritsch & Maniatis, Molecular Cloning: A Laboratory Manual, Second Edition (1989) Cold Spring Harbor Laboratory Press, Cold Spring Harbor, N.Y. (herein "Sambrook et al., 1989"); DNA Cloning: A Practical Approach, Volumes I and II (D. N. Glover ed. 1985); Oligonucleotide Synthesis (M. J. Gait ed. 1984); Nucleic Acid Hybridization [B. D. Hames & S. J. Higgins eds. (1985)]; Transcription And Translation [B. D. Hames & S. J. Higgins, eds. (1984)]; Animal Cell Culture [R. I. Freshney, ed. (1986)]; Immobilized Cells And Enzymes [IRL Press, (1986)]; B. Perbal, A Practical Guide To Molecular Cloning (1984); F. M. Ausubel et al. (eds.), Current Protocols in Molecular Biology, John Wiley & Sons, Inc. (1994).

An embodiment of the invention is a method of identifying an agent that modifies the activity of NAPE-PLD or Abh4, the method comprising: (a) adding NAPE-PLD or Abh4 to at least one receptacle in an array of receptacles; (b) adding an agent to at least one receptacle in the array of receptacles; (c) adding a substrate to at least one receptacle in the array of receptacles wherein the substrate comprises a donor moiety and a quencher moiety wherein intramolecular proximity of the donor moiety and the quencher moiety results in a quenched detectable signal; (d) incubating the agent, the NAPE-PLD or Abh4 and the substrate in the array of receptacles for a predetermined period of time; (e) measuring the amount of detectable signal from the donor moiety following removal of the quencher moiety from the substrate by NAPE-PLD or Abh4 in the array of receptacles; and (f) comparing the amount of detectable signal in a first receptacle comprising the agent, the substrate and the NAPE-PLD or Abh4 to the amount of detectable signal in a second receptacle comprising the substrate and the NAPE-PLD or Abh4 thereby identifying said agent as a modifying agent when the difference exceeds a predetermined threshold.

A further embodiment of the invention is a method of identifying an agent that modifies the activity of NAPE-PLD or Abh4, the method comprising: (a) adding NAPE-PLD or Abh4 to at least one receptacle in an array of receptacles; (b) adding an agent to at least one receptacle in the array of receptacles; (c) adding a substrate to at least one receptacle in the array of receptacles wherein the substrate comprises a donor moiety and a quencher moiety wherein intramolecular proximity of the donor moiety and the quencher moiety results in a quenched detectable signal; (d) measuring the amount of detectable signal from the donor moiety following removal of the quencher moiety from the substrate by NAPE-PLD or Abh4 in the array of receptacles at a first time point; (e) incubating the agent, the NAPE-PLD or Abh4 and the substrate in the array of receptacles for a predetermined period of time; (f) measuring the amount of detectable signal from the donor moiety following removal of the quencher moiety from the substrate by NAPE-PLD or Abh4 in the array of receptacles at a second time point; and (g) comparing the amount of detectable signal at the first time point to the amount of detectable signal at the second time point, thereby identifying said agent as a modifying agent when the difference exceeds a predetermined threshold.

"Predetermined" indicates the period of time or the threshold is established in advance of employing the method of the invention. The period of time or threshold may be selected arbitrarily or empirically. A non-limiting example is to perform a time course and then to select a time to measure the detectable signal based on the results of the time course. "Period of time" can be any unit of time; non-limiting examples include seconds, minutes or hours. "Threshold" refers to the amount of detectable signal required to indicate a response.

An embodiment of the invention utilizes a detectable signal. The skilled artisan can readily appreciate the vast number of detectable signals that may be employed by the instant invention. Non-limiting examples include fluorochromes, chromophores, enzymes, linker moieties, biotin, electron donors, electron acceptors, dyes, metals, and radionuclides.

An embodiment of the invention uses a donor moiety and a quencher moiety. Non-limiting examples include BODIPY and DNP, Rhodamine and DNP, Cy dye and DNP, Alexa and QSY dyes, BODIPY and DNP. The skilled artisan can readily identify other appropriate combinations using different donor and quencher moieties having the appropriate properties such that they may be utilized by the instant invention.

The instant invention is amendable to the utilization of many technologies to generate a detectable signal. A non-limiting example is the use of fluorescence resonance energy transfer (more commonly referred to as FRET). Fluorescence resonance energy transfer is a process whereby the transfer of energy occurs from an excited state fluorophore to a second chromophore in close proximity. For instance, fluorescence resonance energy transfer involves a donor fluorophore in an excited electronic state, which may transfer its excitation energy to a nearby acceptor chromophore through long-range dipole-dipole interactions. Resonance energy transfer can be thought of as coupled oscillators, such as a pair of tuning forks vibrating at the same frequency. Thus, depending on the donor and acceptor moieties used, the skilled artisan can measure the detectable signal resulting from the intramolecular interaction of the donor-acceptor pair and the detectable signal from the donor moiety and the acceptor moiety or both following separation or disruption of the intramolecular interaction. Non limiting examples of donor-acceptor pairs include cyan fluorescent protein-yellow fluorescent protein pair, green fluorescent protein-blue fluorescent protein pairs, europium and allophcocyamin, cyan fluorescent protein/yellow fluorescent protein, green fluorescent protein/blue fluorescent protein, and combination of different Cy dyes, different BODIPY dyes or different Alexa dyes with appropriate excitation and emission wavelengths, Texas Red and Fluorescein, BODIPY and fluorescein, Rhodamine and Fluorescein, combination of different BODIPY dyes with appropriate fluorescent properties, combination of different Cy dyes with appropriate fluorescent properties, and europium chelates or other lanthanide chelates and APC, XL665 or other modified APCs. The skilled artisan can readily appreciate that Cy dye is a general name for many dyes with similar structure but different fluorescent properties. Likewise, BODIPY and Alexa dyes represent a class of dyes that have similar structures and different fluorescent properties. Thus, in theory, a multitude of combinations would be appropriate for FRET. A nonlimiting example is BODIPY 480 with Cy dye 520.

Thus, an embodiment of the invention is a method of identifying an agent that modifies the activity of NAPE-PLD or Abh4, the method comprising: (a) adding NAPE-PLD or Abh4 to at least one receptacle in an array of receptacles; (b) adding an agent to at least one receptacle in the array of receptacles; (c) adding a substrate to at least one receptacle in the array of receptacles wherein the substrate comprises a donor moiety and an acceptor moiety; (d) incubating the agent, the NAPE-PLD or Abh4 and the substrate for a predetermined period of time; (e) measuring the amount of detectable signal wherein the detectable signal measured is selected from the group consisting of the donor moiety, the acceptor moiety, the ratio of donor moiety to acceptor moiety and the donor-acceptor moiety complex; and (f) comparing the amount of detectable signal in a first receptacle comprising the agent, the substrate and the NAPE-PLD or Abh4 to the amount of detectable signal in a second receptacle comprising the substrate and the NAPE-PLD or Abh4 thereby identifying said agent as a modifying agent when the difference exceeds a predetermined threshold.

A further embodiment of the invention is a method of identifying an agent that modifies the activity of NAPE-PLD or Abh4, the method comprising: (a) adding NAPE-PLD or Abh4 to at least one receptacle in an array of receptacles; (b) adding an agent to at least one receptacle in the array of receptacles; (c) adding a substrate to at least one receptacle in the array of receptacles wherein the substrate comprises a donor moiety and an acceptor moiety; (d) measuring the amount of detectable signal at a first time point wherein the detectable signal measured is selected from the group consisting of the donor moiety, the acceptor moiety, the ratio of donor moiety to acceptor moiety and the donor-acceptor moiety complex; (e) incubating the agent, the NAPE-PLD or Abh4 and the substrate for a predetermined period of time; (f) measuring the amount of detectable signal at a second time point wherein the detectable signal measured is selected from the group consisting of the donor moiety, the acceptor moiety, the ratio of donor moiety to acceptor moiety and the donor-acceptor moiety complex; and (g) comparing the amount of detectable signal at the first time point to the amount of detectable signal at the second time point thereby identifying said agent as a modifying agent when the difference exceeds a predetermined threshold.

Since the principle of the instant invention is based on the transfer of energy within the substrate molecule from an energy donor moiety to an energy acceptor moiety. At the broadest scope, the assay is functional when any two suitably situated moieties, the donor and the acceptor (or the quencher), are linked by the substrate molecule. As a non-limiting example, a donor is placed on one of the three fatty acid chains (*e.g*., chain 1 or 2) of the NAPE substrate, and an energy acceptor (or quencher) is placed at a separate site which is still close enough for energy transfer to occur (*e.g*., chain 3, see Figure 3). Before the substrate is hydrolyzed, the donor and acceptor (or quencher) are in close-proximity, and detectable signal from the donor is quenched by the acceptor (or quencher). Upon substrate hydrolysis, the donor and acceptor (or quencher) are separated, resulting in release of the detectable signal, for instance, fluorescence from the donor, that serves as a readout for detecting the enzyme activity. As an alternative example, upon substrate hydrolysis and separation of donor and acceptor, fluorescence from donor can be detected and fluorescence from the acceptor can be detected, wherein one or both of the donor and acceptor signals can be measured or a ratio of donor to acceptor can be measured.

The following are non-limiting examples of combinations of donor-acceptor locations: chain 1 and 3, chain 2 and 3, chain 3 and 1, or chain 3 and 2 (Figure 3). One skilled in the art will appreciate that the hydrocarbon chains of chain 1 and 2 can be varied. Chain 3 may also be varied but the N-acyl moiety should be maintained. The fluorescence probes can be located at any position of the chains as long as the modification does not interfere with the activity of the NAPE-PLD enzyme. The fluorescent donor and acceptor pairs have to be compatible in that, when excited by one wavelength the donor emits light of an appropriate wavelength to be accepted or quenched by the opposing fluorphore. There are several combinations of fluorescent donor-acceptor pairs. As illustration, non-limiting examples of usable combinations include: cyan fluorescent protein-yellow fluorescent protein pair, green fluorescent protein-blue fluorescent protein pairs, europium and allophcocyamin, cyan fluorescent protein/yellow fluorescent protein, green fluorescent protein/blue fluorescent protein, and combination of different Cy dyes, different BODIPY dyes or different Alexa dyes with appropriate excitation and emission wavelengths, BODIPY and DNP, Rhodamine and DNP, Cy dye and DNP, Alexa and QSY dyes, BODIPY and DNP, Texas Red and Fluorescein, BODIPY and fluorescein, Rhodamine and Fluorescein, combination of different BODIPY dyes with appropriate fluorescent properties, combination of different Cy dyes with appropriate fluorescent properties, and europium chelates or other lanthanide chelates and APC, XL665 or other modified APCs.

The skilled artisan can readily appreciate that the order of adding NAPE-PLD or Abh4, agent and substrate can be performed arbitrarily or may be performed together. A non-limiting example is to prepare the NAPE-PLD or Abh4, substrate and agent in one mixture and to add the mixture in one step. Another non-limiting example is to add substrate first, then agent and then NAPE-PLD or Abh4. Any order of addition may be followed. A further embodiment is that an incubation for a predetermined period of time of any one or more of either NAPE-PLD or Abh4, agent or substrate may be added between steps a, b or c. Refinements such as adding the substrate, agent or NAPE-PLD or Abh4 in a single step or in a different order are well within the knowledge and capability of the skilled artisan and are considered embodiments of the invention.

An embodiment of the invention utilizes a substrate. The substrate may be any material or substance that NAPE-PLD or Abh4 may act upon. A non-limiting example is PED6.

An embodiment of the invention utilizes the enzyme NAPE-PLD comprising full-length human NAPE-PLD. NAPE-PLD from other species may also be used. NAPE-PLD may be expressed to comprise other proteins as well, for example, HIS tags that are useful for protein isolation. Further, truncated versions of the NAPE-PLD enzyme may be used. A non-limiting example is SEQ ID NO. 3. If truncated, the truncation may not interfere with the active site. Any truncation may be used provided that the active site is still contained and intact within the truncated protein.

An embodiment of the invention utilizes the enzyme Abh4 comprising full-length human Abh4. A non-limiting example is SEQ ID NO. 5. Abh4 from other species may also be used. Abh4 may be expressed to comprise other proteins as well, for example, HIS tags that are useful for protein isolation. Further, truncated versions of the Abh4 enzyme may be used. If truncated, the truncation may not interfere with the active site. Any truncation may be used provided that the active site is still contained and intact within the truncated protein.

The present invention provides methods for identifying (*e.g*., screening, detecting, characterizing, analyzing and quantifying) agents that modify the activity of NAPE-PLD or Abh4. The term "agent", "test agent", "test compound", "drug candidate" or "modulator" or grammatical equivalents as used herein describes any molecule, either naturally occurring or synthetic, *e.g*., protein, oligopeptide (*e.g*., from about 5 to about 25 amino acids in length, preferably from about 10 to 20 or 12 to 18 amino acids in length, preferably 12, 15, or 18 amino acids in length), small organic molecule, polysaccharide, lipid (*e.g*., a sphingolipid), fatty acid, polynucleotide, oligonucleotide, etc., which is employed in the assays of the invention and assayed for its ability to modulate the activity of NAPE-PLD or Abh4. There are no particular restrictions as to the compound that can be assayed. Examples include single agents or libraries of small, medium or high molecular weight chemical molecules, purified proteins, expression products of gene libraries, synthetic peptide libraries, cell extracts and culture supernatants. An agent encompasses any combination of different agents.

An agent may include at least one or more soluble or insoluble factors, cell matrix components, conditioned media, cell extracts, tissue extracts, explants, pH modifiers, gasses, osmotic pressure modifiers, ionic strength modifiers, viruses, DNA, RNA or gene fragments. An agent can be in the form of a library of test agents, such as a combinatorial or randomized library that provides a sufficient range of diversity or conversely is limited to similar structures or features. Agents can be optionally linked to a fusion partner, *e.g*., targeting compounds, rescue compounds, dimerization compounds, stabilizing compounds, addressable compounds, and other functional moieties. Conventionally, new chemical entities with useful properties are generated by identifying a test agent (called a "lead compound" or a "lead") with some desirable property or activity, e.g., inhibiting activity or modulating activity. The lead compound is then used as a scaffold to create variants of the lead compound, and further evaluate the property and activity of those variant compounds.

An agent may include treatment conditions and manipulation of external and internal conditions or environment. A non-limiting example of such an agent includes ultraviolet light.

An embodiment of the invention is a method for identifying an agent that modifies the activity of NAPE-PLD or Abh4. The term "modifies" encompasses any change in activity or specificity of NAPE-PLD or Abh4. Thus, the term "modifies" may refer to an agent that is an inhibitor, inducer or modulator of NAPE-PLD activity or Abh4 activity.

The term "inhibitor" encompasses any drug, chemical, protein or protein fragment capable of blocking, interrupting or preventing the activity or specificity of NAPE-PLD or Abh4. Further, an inhibitor may be, *e.g*., a molecular chaperone, antibody or inhibitory RNA (RNAi) that blocks expression of cellular proteins thereby inhibiting pathways directly or indirectly affecting NAPE-PLD or Abh4. "Block", "blocks" or "blocking" need not be absolute but any measurable inhibition is an indication that blocking has occurred. An "inhibitor" may be the manipulation of culturing conditions such as oxygen augmentation or deprivation or changing media components in such a manner as to block, interrupt or prevent a cellular response thereby preventing or interrupting, or blocking the activity or specificity of NAPE-PLD or Abh4.

The term "inducer" encompasses any drug, chemical, protein or protein fragment capable of initiating, stimulating or enhancing the activity or specificity of NAPE-PLD or Abh4. Further, an inducer may be a molecular chaperone, antibody or inhibitory RNA (RNAi) that blocks expression of cellular proteins thereby removing inhibition or directly initiating or stimulating a cellular response, activity or pathway. An "inducer" may be the manipulation of culturing conditions such as oxygen augmentation or deprivation or changing media components in such a manner as to block, interrupt or prevent a cellular response ultimately initiating, stimulating or enhancing the activity or specificity of NAPE-PLD or Abh4.

The term "modulator" encompasses any drug, chemical, protein or protein fragment capable of adjusting the intensity, proportion or the characteristics of the activity or specificity of NAPE-PLD or Abh4 or of a cellular response, activity or pathway involved in the activity or specificity of NAPE-PLD or Abh4. Further, a modulator may be a molecular chaperone, antibody or inhibitory RNA (RNAi) that blocks expression of cellular proteins thereby removing inhibition or inducing a cellular response, activity or pathway that ultimately affects the activity or specificity of NAPE-PLD or Abh4. A "modulator" may be the manipulation of culturing conditions such as oxygen augmentation or deprivation or changing media components in such a manner as to adjust the intensity, proportion or the characteristics of a cellular response that may affect the activity or specificity of NAPE-PLD or Abh4.

In a further embodiment of the invention, a modulator may be used in conjunction with an inducer such that a modulator of an inducer makes the inducer more potent (*e.g*., resulting in an enhanced NAPE-PLD or Abh4 activity) or the inducer less potent (*e.g*., resulting in a reduced NAPE-PLD or Abh4 activity). A modulator of an inhibitor makes the inhibitor less potent (*e.g*., enhanced NAPE-PLD or Abh4 activity) or the inhibitor more potent (*e.g*., reduced NAPE-PLD or Abh4 activity).

Inhibitors, inducers or modulators can also be utilized to mimic pathways or aspects of disease states. As a non-limiting illustrative example, an embodiment of the invention would be to identify an agent that modifies the activity of NAPE-PLD or Abh4 in the presence of an NAPE-PLD or Abh4 inhibitor, inducer or modulator that mimics aspects of a psychological disorder, diabetes or obesity. Thus, an embodiment of the invention encompasses a screening method for identifying a test agent that may ameliorate a disease state where NAPE-PLD or Abh4 activity or specificity is thought to be affected.

Another embodiment of the invention is the use of more than one inducer, inhibitor or modulator. Using more than one inducer, inhibitor or modulator could, but is not limited to, having additive effects, counter effects, synergistic effects or affecting multiple pathways.

An embodiment of the invention is a method of identifying an agent that modifies the activity of NAPE-PLD or Abh4, the method further comprising a control. A control is a term of art well understood by skilled artisans. An appropriate control may be dependent on the assay parameters utilized or the experimental question under investigation. A control may be a particular set of assay conditions or the addition or elimination of a particular compound to the culture medium. A control may be considered a positive control in that the assay conditions or control compound added brings about the anticipated response. For example, if the agent under investigation is expected to inhibit NAPE-PLD or Abh4, a positive control would be a compound or agent known to inhibit NAPE-PLD or Abh4. A control may also be a negative control. A negative control may be a particular set of assay conditions or the addition or elimination of a particular compound or agent to the culture medium that would bring about the anticipated response. For example, if the agent under investigation is expected to inhibit NAPE-PLD or Abh4, then a negative control would be expected to not to inhibit NAPE-PLD or Abh4. A control may be a "vehicle" control. For example, if the test agent is dissolved in DMSO then the vehicle control would be DMSO without test agent. A control may simply be the use of historical data.

An embodiment of the invention is a method wherein the NAPE-PLD or Abh4 is provided by culturing in the receptacles a prokaryotic or eukaryotic cell that is transiently or stably transfected to express the NAPE-PLD or Abh4. Thus, a further embodiment of the invention is to use cells transiently or stably transformed to express or overexpress NAPE-PLD or Abh4. Expression can be induced or constitutive. Agents can be tested for their ability to modulate NAPE-PLD or Abh4 activity by adding the agent to receptacles comprising cultured cells transiently or stably transformed to express or overexpress NAPE-PLD or Abh4.

A recombinant expression vector of the invention comprises a nucleic acid molecule in a form suitable for expression of the nucleic acid in a host cell. Thus, a recombinant expression vector of the present invention can include one or more regulatory sequences, selected on the basis of the host cells to be used for expression, that is operably linked to the nucleic acid to be expressed. Within a recombinant expression vector, "operably linked" is intended to mean that the nucleotide sequence of interest is linked to the regulatory sequence(s) in a manner that allows for expression of the nucleotide sequence (e.g., in an in vitro transcription/translation system or in a host cell when the vector is introduced into the host cell). The term "regulatory sequence" is intended to include promoters, enhancers and other expression control elements (e.g., polyadenylation signals). Such regulatory sequences are described, for example, in Goeddel, Gene Expression Technology: Methods in Enzymology Vol. 185, Academic Press, San Diego, Calif. (1990). Regulatory sequences include those that direct constitutive expression of the nucleotide sequence in many types of host cells (e.g., tissue specific regulatory sequences). It will be appreciated by those skilled in the art that the design of the expression vector can depend on such factors as the choice of host cell to be transformed, the level of expression of protein desired, etc. The expression vectors of the invention can be introduced into host cells to produce proteins or peptides encoded by nucleic acids as described herein.

The term "overexpression" as used herein, refers to the expression of a polypeptide, e.g., a molecule that may be involved in apoptosis or cell survival mechanisms, by a cell, at a level that is greater than the normal level of expression of the polypeptide in a cell that normally expresses the polypeptide or in a cell that does not normally express the polypeptide. For example, expression of the polypeptide may by 10%, 20%, 30%, 40%, 50%, 60%, 70, 80%, 90%, 100%, or more as compared to expression of the polypeptide in a wild-type, ground state or uninduced cell that normally expresses the polypeptide. Mutants, variants, or analogs of the polypeptide of interest may be overexpressed.

As used herein, the term "transient" expression refers to expression of exogenous nucleic acid molecule(s) which are separate from the chromosomes of the cell. Transient expression generally reaches its maximum 2-3 days after introduction of the exogenous nucleic acid and subsequently declines.

As used here, the term "stable" expression refers to expression of exogenous nucleic acid molecule(s) that are part of the chromosomes of the cell. In general, vectors for stable expression of genes include one or more selection markers.

Cell culturing techniques for transformed, non-transformed, primary culture and biological samples are well known in the art. Biological samples or cultured cells can be stored until required for use. The media used for culturing can be specifically designed or purchased from commercial sources.

An embodiment of the invention uses cell lines that are commercially available. For example, cells that can be used are available from the American Tissue Culture Company or other sources. Cells may be prokaryotic or eukaryotic. The invention is not limited by the type of cells used. Primary cultures may also be utilized. Non-differentiated cells may be subjected to various agents to cause the cells to differentiate into a particular phenotype. For example, progenitor cells induced to differentiate into oligodendrocytes would be an embodiment of the invention. The particular cell type used may be selected by markers specifically expressed by the desired cell type, or alternatively, by the loss of a particular marker(s). Cells can be separated or sorted by methods such as flow cytometry that are commonly used by skilled artisans.

An embodiment of the invention uses a homogeneous cell population. An alternative embodiment of the invention uses a heterogeneous cell population. The cells can be of any type and in any proportion to complete the assay of the invention.

Cells may be obtained from a biological sample. A biological sample may include, but is not limited to, tissue or fluids, sections of tissues such as biopsy and autopsy samples, and frozen sections taken for histologic purposes. Such samples include blood, sputum, tissue, cultured cells, *e.g*., primary cultures, explants, and transformed cells, stool, urine, etc. A biological sample can be obtained from a eukaryotic organism, including from mammals such as a primate, *e.g*., chimpanzee, macaque or human, cow, dog, cat, a rodent, *e.g*., guinea pig, rat, mouse, rabbit, or a bird, reptile, or fish.

An embodiment of the invention is use in high throughput screening (HTS) methods. HTS is the automated, simultaneous testing of thousands of distinct chemical compounds in assays designed to model biological mechanisms or aspects of disease pathologies. More than one compound, *e.g*., a plurality of compounds, can be tested simultaneously, *e.g*., in one batch. In one embodiment, the term HTS screening method refers to assays which test the ability of one compound or a plurality of compounds to influence the readout of choice.

An embodiment of the invention utilizes robotic or workstation device. Liquid handling systems, analytical equipment such as fluorescence readers or scintillation counters and robotics for cell culture and sample manipulation are well known in the art. Mechanical systems such as robotic arms or "cherry-picking" devices are available to the skilled artisan. Commercial plate readers are available to analyze, *e.g*., conventional 96-well or 384-well plates. Single sample, multiple sample or plate sample readers are available that analyze predetermined wells and generate raw data reports. The raw data can be transformed and presented in a variety of ways.

An embodiment of the invention comprises an array of receptacles that can receive cells and other materials such as culture media. An array of receptacles can be any number of receptacles from at least one or more than one receptacle suitable for holding cells within the scope of the invention. Examples include but are not limited to flasks, culture dishes, tubes such as 1.5 ml tubes, 12 well plates, 96 well plates, 384 well plates, 1536 well plates and miniaturized microtiter plates with perhaps 4000 receptacles (U.S. Patent Application 20050255580). The array of receptacles may be amendable to the addition of a protective covering thus preventing against entry of contaminants or evaporation of contents.

A further characteristic of the receptacles is that the receptacle may allow for analysis, non-limiting examples include, spectrophotometric analysis, scintillation counting and fluorescence measurements. However, this is not a limitation to receptacles that can be used within the scope of the invention given that samples can be transferred to a suitable container amenable to further analysis. A non limiting example is to modify the method such that the method further comprises providing a second array of receptacles wherein samples from the first array of receptacles is transferred to the second array of receptacles.

An embodiment of the invention is an assay system for identifying an agent that modifies the activity of NAPE-PLD or Abh4, the assay system comprising: (a) an array of receptacles; (b) NAPE-PLD or Abh4; (c) a substrate comprising a donor moiety and a quencher moiety wherein the donor moiety is capable of detection; and (d) at least one component wherein the component is selected from the group consisting of the agent(s), an inducer(s), an inhibitor, a modulator(s), a modulator(s) of the inducer(s), a modulator(s) of the inhibitor(s) and control(s).

Another embodiment of the invention is an assay system for identifying an agent that modifies the activity of NAPE-PLD or Abh4, the assay system comprising: (a) an array of receptacles; (b) NAPE-PLD or Abh4; (c) a substrate comprising a donor moiety and an acceptor moiety wherein the donor moiety or the acceptor acceptor moiety or both are capable of detection; and (d) at least one component wherein the component is selected from the group consisting of the agent(s), an inducer(s), an inhibitor, a modulator(s), a modulator(s) of the inducer(s), a modulator(s) of the inhibitor(s) and control(s).

An embodiment of the invention is a component that modifies the activity or specificity of NAPE-PLD or Abh4. The component is selected from the group consisting of a control, an agent, an inducer, an inhibitor, a modulator, a modulator of the inducer and a modulator of the inhibitor. The assay system can have more than one component.

A further embodiment of the invention is a kit comprising at least one element of the assay system and instructions for use. Thus, the components of the assay system may be provided separately or may be provided together such as in a kit. Components of the assay system may be prepared and included in a kit according to methods that maximize the stability of the individual components. Such methods are familiar to those persons skilled in the art. For example, cells of the assay system may be provided as a suspension or lyophilized. Additional components of the system may also be included such as buffers, containers for mixing the assay components such as microtiter plates or test tubes. The assay system can be provided in the form of a kit that includes instructions for performing the assay and instructions for data handling and interpretation.

An embodiment of the invention is a pharmaceutical composition for the modulation of NAPE-PLD or Abh4 comprising a therapeutically effective amount of an agent identified by the methods of the invention and a pharmaceutically acceptable carrier. The term "therapeutically effective amount" refers to an amount of an agent effective to treat a disease or disorder in at subject or mammal. As a non-limiting example, in the case of obesity, the therapeutically effective amount of the drug may reduce the body weight. The pharmaceutical compositions of the present invention can be used in combination with other therapeutic agents. For example, in the treatment of obesity, the pharmaceutical composition may be given in combination with other appetite suppressants.

The invention is further described in the following examples, which do not limit the scope of the invention described in the claims. While the invention has been described and exemplified in sufficient detail for those skilled in this art to produce and use it, various alternatives, modifications, and improvements should be apparent without departing from the scope of the claimed invention. One skilled in the art readily appreciates that the present invention is well adapted to carry out the objective and obtain the ends and advantages mentioned, as well as those inherent therein. The examples that follow are descriptions of embodiments and are not intended as limitations on the scope of the invention. Modifications therein and other uses will occur to those skilled in the art. These modifications

The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed.

### Example 1

### PREPARATION OF NAPE-PLD ENZYME:

NAPE-PLD has been expressed in E.coli and mammalian cells. Mammalian expression systems were not a good choice for because of the relatively low activity of NAPE-PLD and the large quantity of protein needed for high throughput screening. In order to obtain large amount of protein to support HTS, we tested the expression of NAPE-PLD in E.coli and baculovirus/insect cell systems. The NAPE-PLD expressed in E.coli has lower solubility, lower specific activity, and lower expression level than the protein expressed in baculovirus/insect cells. So, we selected the baculovirus/insect cells as the expression system for supporting HTS. The pDEST8.1 vector (Invitrogen) was used to express NAPE-PLD with a C-terminal 6x His tag (SEQ ID NO. 1) in insect cells. This expression system made it possible to generate a large quantity of NAPE-PLD with high purity through metal chelating chromatography.

The sequence of NAPE-PLD has been previously described (SEQ ID NO. 2). The protein with C-terminal His tag (MW 49 kDa) was expressed and purified In soluble, active form from insect cells in the presence of Triton X-100. The expressed enzyme was found to be truncated at the N-terminus by 46 amino acids (SEQ ID NO. 3).

### Example 2

### GENERAL ASSAY PARAMETERS:

General assay conditions use the following final concentrations of the following material: NAPE-PLD at 14 nM, PED6 (substrate) at 10 µM, test compounds at 10 µM, DMSO at 1 %. PED6 is purchased (see Table 1) as a lyophilized powder and stored at -20 °C. A fresh substrate solution should be prepared daily. 1mg of PED6 is dissolved in 0.88 ml of ethyl acetate and diluted into 43.12 ml assay buffer to make a 20 µM working solutions. PED6 must be kept out of direct light, as it is light sensitive.

**Table 1:**

| **Materials** | **Supplier** | **Catalog Number** | **Function** |
|---|---|---|---|
| Black 384 square well, | Costar | 3654 | Assay Plate |
| polystyrene non- | | | |
| binding surface assay | | | |
| plates | | | |
| LJL Acquest | Molecular Device | | Assay Plate Reader |
| CyBi-Well Automated | Cy-Bio | Serial # 3191-3- | Compound addition |
| CyBi-CyBi-Well | | 3122 | |
| Automated | | | |
| Workstation | | | |
| FLEXDROP 384 | Perkin Elmer | Model # AFELV40 | Bulk reagents |
| Automatic Dispenser | | | dispenser |
| PED6 | Invitrogen | D23739 | Substrate |
| Triton X-100, | CalBiochem | 648464 | Detergent |
| hydrogenated | | | |
| 10% Solution | | | |

Test compounds are diluted in an appropriate buffer. A non-limiting example is 50 mM Tris-HCl, pH 8.0, the buffer may contain DMSO, for instance, 10% DMSO. Assay buffer was 50 mM Tris-HCl, pH 8.0, 0.05% Triton X-100 and stop solution is 0.4 M HCl , 0.05% Triton X-100.

Order of addition of reagents and equipment used:
- 1): Dilute enzyme (NAPE-PLD, 22 µM) to 28 nM in assay buffer.
- 2): Prepare substrate (20 µM in assay buffer).
- 3): Add 10 µl NAPE-PLD to assay plate using FLEXDROP.
- 4): Add 2 µl compound (from compound plate) to appropriate wells of assay plate using Cy-Bio.
- 5): Incubate plates, covered, at room temperature for 30 minutes (compound preincubation).
- 6): Add 10 µl PED6 to assay plate using FLEXDROP.
- 7): Incubate plates, covered, at room temperature for 30 minutes (enzyme reaction)
- 8): Terminate the reaction by adding 20 µl of stop solution, using FLEXDROP
- 9): Read on LJL Acquest. Excitation filter, 485 nM; emission filter, 530 nM.

### Example 3

### OPTIMIZATION OF SUBSTRATE (PED6) SOLVENT:

Since PED6 and other potential substrates of NAPE-PLD are lipids, they are extremely hydrophobic. Therefore, it is reasonable to dissolve the substrate in chloroform before adding aqueous assay buffer. We added 0 - 0.05% of Triton X-100 in order to further increase the solubility of PED6 in aqueous solution. Further, high concentration of Triton X-100 was also reported to increase the activity of NAPE-PLD (up to 0.2%) (Petersen and Hansen, FEBS Lett. 455:41-44, 1999). Under these substrate solvent conditions, the assay did not work (see Table 2).

**Table 2. NAPE-PLD Substrate Assay Conditions**

| **Condition** | **Organic Solvent** **Used to Initially** **Dissolve PED6** | **Final Detergent** **Concentration in** **Assay Buffer** | **Lipids Used to** **Aid PED6** **Solubility** | **NAPE-PLD Activity** |
|---|---|---|---|---|
| 1 | Chloroform used to dissolve PED6 initially, then dried down before adding aqueous | 0 - 0.05% Triton X- 100 | No | No |
| 2 | Chloroform | none | PI/PC ratio from 1:1 to 3:1 | No |
| 3 | Chloroform | 0 - 0.05% Triton X- 100 | PI/PC ratio from 1:1 to 3:1 | No |
| 4 | Ethyl Acetate* | 0 - 0.015% Triton X-100 | No | No |
| 5 | Ethyl Acetate * | > 0.015 - 0.1% Triton X-100 | No | Yes |
| 6 | Ethyl Acetate | >0.1% Triton X- 100 | No | No |
| *(final concentration in assay buffer 1%) | | | | |
| PC=egg yolk phosphatidylcholine, PI=soybean phosphatidylinositol | | | | |
| Total PI + PC = 6 mg/ml | | | | |

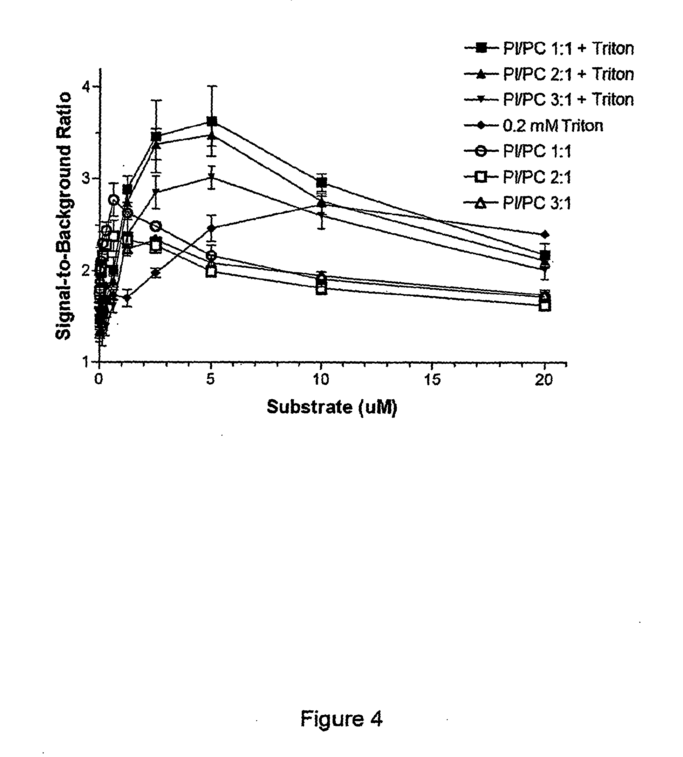

Further research into the problem of how to properly prepare the substrate led us to investigate the assay conditions for other PLDs. One paper used hydrophobic lipid substrates of PLDs that were dissolved in chloroform and were then mixed with a phosphatidylinositol/phosphatidylcholine (PI/PC) blend dissolved in chloroform (Petry et al., J. Lipid Res. 46:603-614, 2005). The final mixture was then dried. To resuspend the mixture in aqueous buffer, ultrasonic treatment was needed. Following this procedure, the assay started to provide a detectable signal, but the signal to background ratio was low. Adding Triton X-100 to the aqueous assay buffer increased the signal to background ratio, but also increased the assay noise (Figure 4). Such a noisy assay was not suitable for high throughput screening.

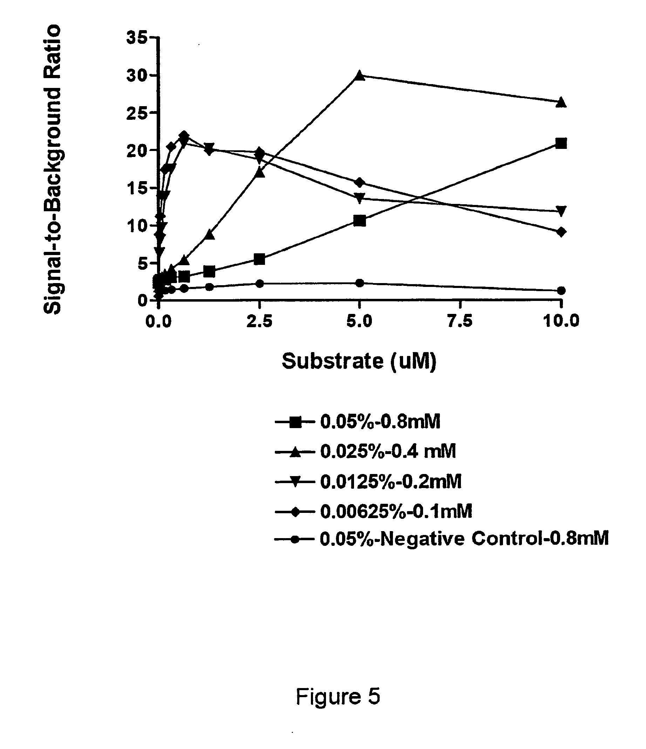

There are three ways to increase the solubility of hydrophobic lipid substrates in aqueous buffers: organic solvent (like chloroform), lipids (like PI and PC), or detergents (like Triton X-100). Since the organic solvent chloroform used in the initial dissolution ultimately damages the protein, we had to dry off the chloroform before adding aqueous buffer. We hypothesized that if we used a more "protein friendly" organic solvent, such as ethyl acetate, it could remain in the aqueous assay buffer without damaging the protein and help to increase the solubility of the hydrophobic lipid substrates. Based on this theory, we used ethyl acetate to dissolve the hydrophobic lipid substrate, then added aqueous buffer containing 0-0.1% Triton X-100 without drying off the ethyl acetate. This assay strategy worked well (see Table 2). However, the signal-to-background detection window for Triton X-100 working concentrations was limited. Under low Triton X-100 concentrations, the hydrophobic lipid substrate was not fully soluble and was not available to the NAPE-PLD enzyme, therefore, the signal-to-background ratio was small. Increasing the concentration of Triton X-100 increased the availability of the substrate to the enzyme, resulted in higher signal-to-background ratio. However, further increasing Triton X-100 concentration resulted in much higher background fluorescence, thus reducing the signal-to-background ratio (Figure 5). Thus, for optimal substrate preparation conditions we selected condition 5 (Table 2).

### Example 4

### SUBSTRATE AND ENZYME TITRATION:

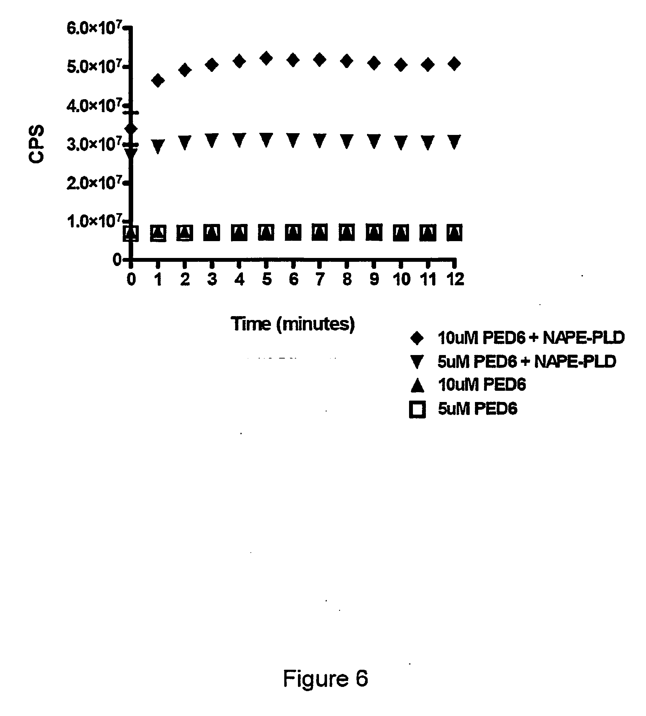

For substrate titration, as the substrate concentration was increased from 5 µM to 10 µM, the detection window was almost doubled while the background signal remained constant (Figure 6). A high concentration of enzyme (1:100) was used in order to find the maximum fluorescence signal under current buffer conditions (0.05% Triton X-100).

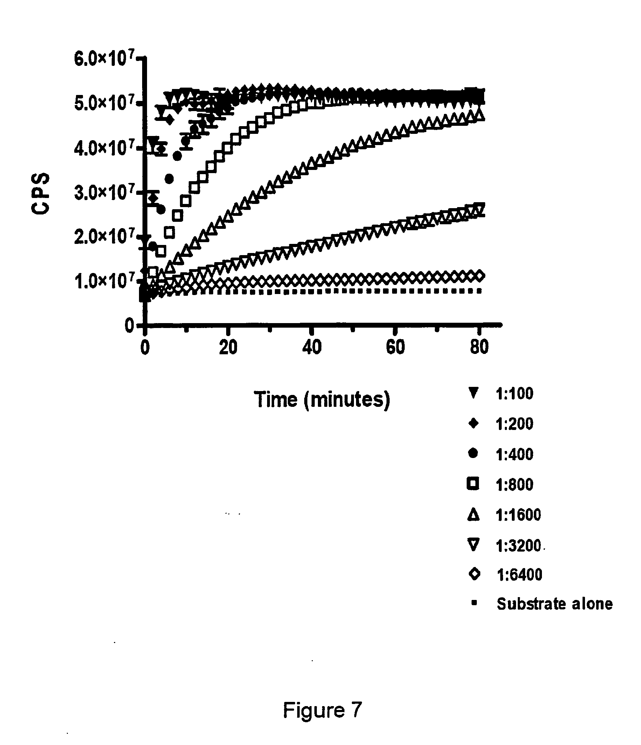

For enzyme titration, NAPE-PLD was serially diluted from 1:100 up to 1:6400 (Figure 7). The reaction was started by adding PED6 to a final concentration of 10 µM. The reaction volumes were 20 µl. The fluorescent intensity was measured every two minutes. Based on these results, 14 nM enzyme (1:1600) and a reaction time of 30 min were selected for the further experiments.

### Example 5

### Km DETERMINATION FOR NAPE-PLD:

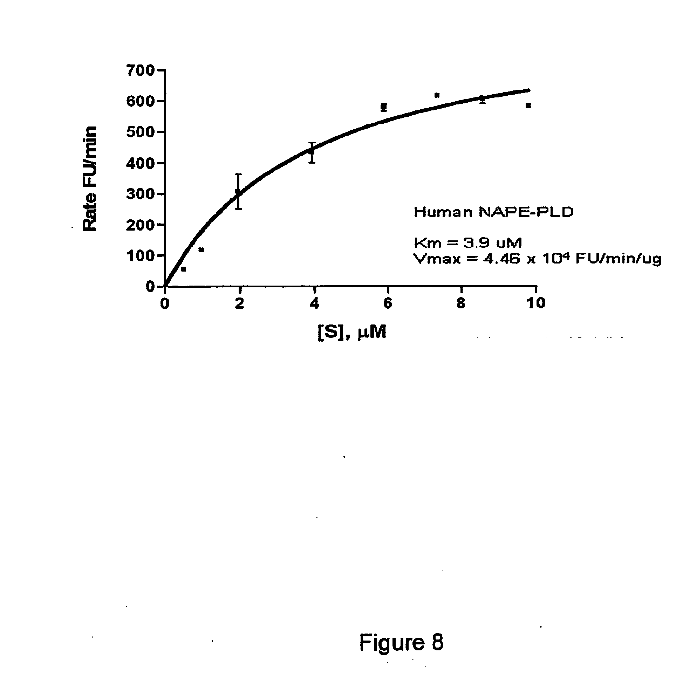

Values for Kₘ of 3.9 µM and Vmax of 4.46 x10⁴ FU/min/□g protein (Figure 8) were determined. The Vmax value was normalized for the amount of protein used. In order to be able to identify inhibitors with different mode of actions, including competitive inhibitors, we chose to start the substrate titration from a concentration of 5 µM, a concentration close to the determined Km value, and found 10 µM substrate concentration gave the best combination of signal to background ratio and sensitivity to inhibitor. Further, the following parameters for the NAPE-PLD assay conditions: 14 nM enzyme, 10 µM substrate with a reaction time of 30 minutes in a 20 µl volume.

### Example 6

### DMSO SENSITIVITY:

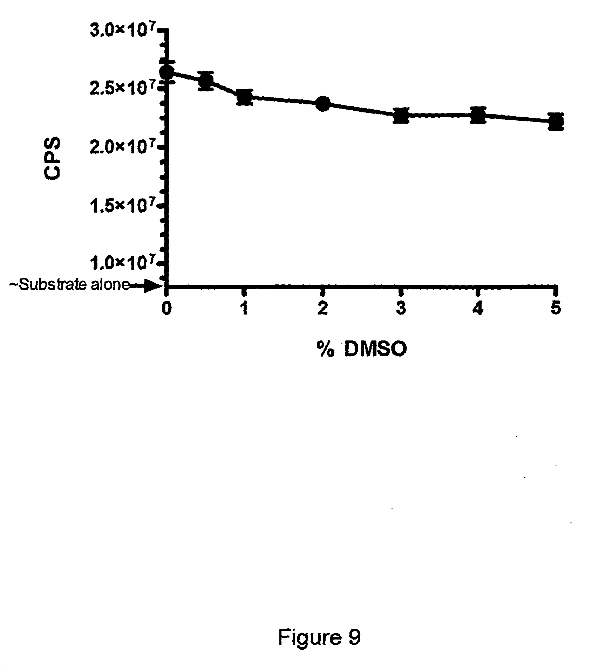

Since many chemical libraries are kept as stock solutions in DMSO, we tested the sensitivity of the assay to DMSO (Figure 9). The assay was somewhat sensitive to DMSO but DMSO was well tolerated.

### Example 7

### REPRESENTATIVE IC₅₀ CURVES:

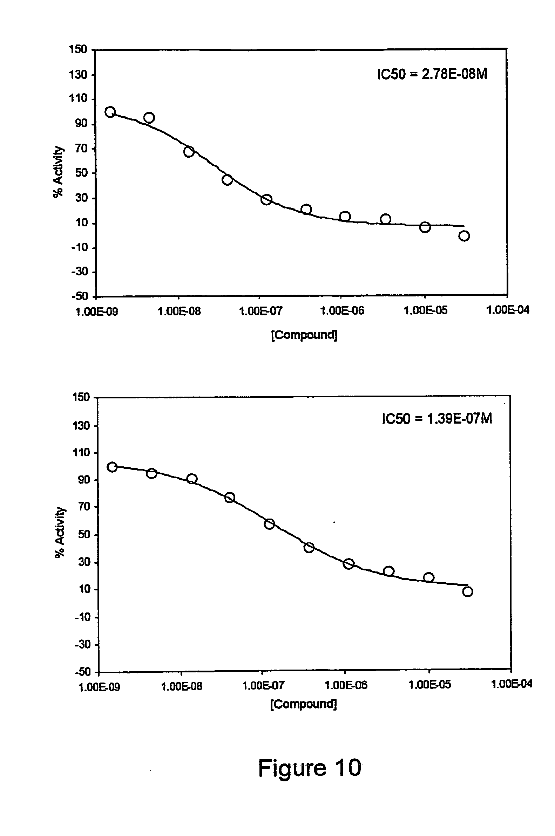

There are no known inhibitors of NAPE-PLD. Therefore, compounds that were found to inhibit NAPE-PLD during validation of the assay were used to demonstrate dose response activity in the assay (Figure 10). It is expected that a common "false positive" effect in the primary assay will be due to micelle-disrupting compounds, since the hydrophobic substrate is very dependent on the detergent in the buffer for solubility. When Triton X-100 is not present in the assay buffer, the substrate fluorescence is much lower, and this effect could mimic a positive compound. To identify such "false positives", one can measure fluorescence in the presence of compound and substrate, but in the absence of enzyme, to determine if compound interferes with the fluorescent intensity of the substrate in the presence of Triton X-100.

### Example 8

### PREPARATION OF Abh4 AND TESTING OF PED6 AS SUBSTRATE

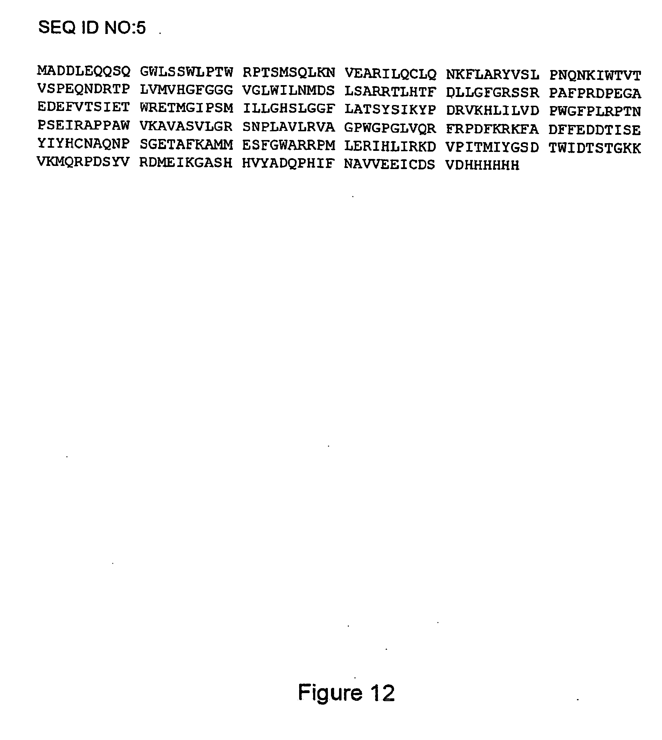

Human Abh4 was cloned and expressed in FreeStyle 293F cells (Invitrogen), a suspension cell line for transient expression, using the pcDNA3.1zeo_DEST vector. The pcDNA3.1zeo_DEST is a modified vector of pcDNA3.2zeo (Invitrogen). The pcDNA3.1zeo_DEST vector containing the Abh4 coding sequences is disclosed in SEQ ID NO: 4. Abh4 was expressed with a C-terminal His tag (SEQ ID NO: 5). A portion of the crude lysate was tested for hydrolysis of PED6 and resulting fluorescence. Lysate (75 µg protein) from transfected cells was added to a solution of PED6 (5 µM final assay concentration) in Tris HCl buffer (pH 7.6) containing 0.05% Triton X-100 (to solubilize the substrate) in final volume of 100 µL. The reaction in a 96-well black plate was monitored continuously in a fluorescence plate reader Gemini, (SpectraMax), with excitation and reading from the top (excitation wavelength 488 nm, emission wavelength 520 nm). Lysate (75 µg protein) of FreeStyle 293F cells that had not been transfected was used as a control. Under these conditions, a 19-fold higher rate was observed with the lysate from transfected cells over the control lysate, suggesting that the Abh4 present is efficiently hydrolyzing the substrate PED6 (Figure 12). To further validate this finding, the inhibitor MAFP (methylarachidonylfluorophosphonate) was used. At a concentration of 5.34 µM, this inhibitor showed >95% inhibition of the activity observed, consistent with the findings of Simon and Cravatt (2006).

Thin layer chromatography (TLC) was performed to establish that the products of hydrolysis of PED6 by Abh4 and by NAPE-PLD are different. TLC on silica gel using chloroform:methanol:ammonia system of ethyl acetate-extracted enzymatic reaction mixture showed that the reaction products are different.

## Claims

1. A method of identifying an agent that modifies the activity of N-acylphosphatidylethanolamine-hydrolysing phospholipase D (NAPE-PLD) or alpha/beta-hydrolase-4 (Abh4), the method comprising:
a) adding NAPE-PLD or Abh4 to at least one receptacle in an array of receptacles;
b) adding an agent to at least one receptacle in the array of receptacles;
c) adding a substrate to at least one receptacle in the array of receptacles wherein the substrate comprises a donor moiety and a quencher moiety wherein intramolecular proximity of the donor moiety and the quencher moiety results in a quenched detectable signal;
d) incubating the agent, the NAPE-PLD or Abh4 and the substrate in the array of receptacles for a predetermined period of time;
e) measuring the amount of detectable signal from the donor moiety following removal of the quencher moiety from the substrate by NAPE-PLD or Abh4 in the array of receptacles; and
f) comparing the amount of detectable signal in a first receptacle comprising the agent, the substrate and the NAPE-PLD or Abh4 to the amount of detectable signal in a second receptacle comprising the substrate and the NAPE-PLD or Abh4
thereby identifying said agent as a modifying agent when the difference exceeds a predetermined threshold
wherein the donor moiety and the quencher moiety are selected from the group consisting of BODIPY/DNP, Rhodamine/DNP and Cy dye/DNP.

2. A method according to claim 1 wherein steps (d) to (f) are replaced by the following steps (d) to (g);
d) measuring the amount of detectable signal from the donor moiety following removal of the quencher moiety from the substrate by NAPE-PLD or Abh4 in the array of receptacles at a first time point;
e) incubating the agent, the NAPE-PLD or Abh4 and the substrate in the array of receptacles for a predetermined period of time;
f) measuring the amount of detectable signal from the donor moiety following removal of the quencher moiety from the substrate by NAPE-PLD or Abh4 in the array of receptacles at a second time point; and
g) comparing the amount of detectable signal at the first time point to the amount of detectable signal at the second time point,
thereby identifying said agent as a modifying agent when the difference exceeds a predetermined threshold.

3. A method of identifying an agent that modifies the activity of N-acylphosphatidylethanolamine-hydrolysing phospholipase D (NAPE-PLD) or alpha/beta-hydrolase-4 (Abh4), the method comprising:
a) adding NAPE-PLD or Abh4 to at least one receptacle in an array of receptacles;
b) adding an agent to at least one receptacle in the array of receptacles;
c) adding a substrate to at least one receptacle in the array of receptacles wherein the substrate comprises a donor moiety and an acceptor moiety;
d) incubating the agent, the NAPE-PLD or Abh4 and the substrate for a predetermined period of time;
e) measuring the amount of detectable signal wherein the detectable signal measured is selected from the group consisting of the donor moiety, the acceptor moiety, the ratio of donor moiety to acceptor moiety and the donor-acceptor moiety complex; and
f) comparing the amount of detectable signal in a first receptacle comprising the agent, the substrate and the NAPE-PLD or Abh4 to the amount of detectable signal in a second receptacle comprising the substrate and the NAPE-PLD or Abh4
thereby identifying said agent as a modifying agent when the difference exceeds a predetermined threshold
wherein the donor moiety and the acceptor moiety are selected from the group consisting of Texas red and fluorescein, BODIPY and fluorescein, Europium and allophcocyamin (APC), cyan fluorescent protein/yellow fluorescent protein, and green fluorescent protein/blue fluorescent protein.

4. A method according to claim 3 wherein steps (c) to (f) are replaced by steps (c) to (g) as follows:
c) adding a substrate to at least one receptacle in the array of receptacles
wherein the substrate comprises a donor moiety and an acceptor moiety;
d) measuring the amount of detectable signal at a first time point wherein the detectable signal measured is selected from the group consisting of the donor moiety, the acceptor moiety, the ratio of donor moiety to acceptor moiety and the donor-acceptor moiety complex;
e) incubating the agent, the NAPE-PLD or Abh4 and the substrate for a predetermined period of time;
f) measuring the amount of detectable signal at a second time point wherein the detectable signal measured is selected from the group consisting of the donor moiety, the acceptor moiety, the ratio of donor moiety to acceptor moiety and the donor-acceptor moiety complex; and
g) comparing the amount of detectable signal at the first time point to the
amount of detectable signal at the second time point thereby identifying said agent as a modifying agent when the difference exceeds a predetermined threshold.

5. The method of claim 3 or 4 wherein the detectable signal is measured by fluorescence resonance energy transfer.

6. The method of claim 1,2,3, or 4 further comprising incubation of the agent and the NAPE-PLD or Abh4 for a predetermined period of time before step c.

7. The method of claim 1, 2, 3 or 4 wherein steps a, b and c can be performed in any order or can be performed together.

8. The method of claim 1, 2, 3 or 4 further comprising at least one control.

9. The method of claim 1, 2, 3 or 4 further comprising at least one inhibitor, Inducer or modulator.

10. The method of claim 1, 2, 3 or 4 wherein the substrate is PED6.

11. The method of claim 10 wherein the PED6 is dissolved in ethyl acetate.

12. The method of claim 11 wherein the dissolved PED6 is added to a buffer comprising about 0.005 to 0.1 % Triton X-100.

13. The method of 12 wherein the buffer comprises about 0.05 % Triton X-100.

14. The method of claim 1, 2, 3 or 4 wherein the detectable signal is selected from the group consisting of a fluorochrome, chromophore, enzyme, linker moiety, biotin, electron donors, electron acceptors, dyes, metals, and radionuclide.

15. The method of claim 1, 2, 3 or 4 wherein the NAPE-PLD or Abh4 in step a is provided by culturing in the receptacle a prokaryotic or eukaryotic cell that is transiently or stably transfected to express the NAPE-PLD or Abh4.

16. The method of claim 1, 2, 3 or 4 wherein NAPE-PLD or Abh44 is provided following isolation from a biological sample or from cells transiently or stably transfected to express NAPE-PLD or Abh4.

17. The method of claim 18 wherein the biological sample is from a mammal selected from the group consisting of human, monkey, rat, mouse, rabbit, and guinea pig.

18. The method of claim 1, 2, 3 or 4 wherein one or more steps are performed by a robotic or workstation device.

19. An assay system for identifying an agent that modifies the activity of NAPE-PLD or Abh4, the assay system comprising:
a) an array of receptacles;
b) NAPE-PLD or Abh4; and
c) a substrate comprising a fluorescent donor moiety and a quencher moiety wherein the donor moiety is capable of detection

20. An assay system for identifying an agent that modifies the activity of NAPE-PLD or Abh4, the assay system comprising:
a) an array of receptacles;
b) NAPE-PLD or Abh4; and
c) a substrate comprising a fluorescent donor moiety and an acceptor moiety wherein the donor moiety or the acceptor moiety or both are capable of detection.

## Patentansprüche

1. Verfahren zur Erkennung Wirkstoffes, welcher die Aktivität von N-Acylphosphatidylethanolaminhydrolysierender Phospholipase D (NAPE-PLD) oder alpha/beta-Hydrolase-4 (Abh4) modifiziert, wobei das Verfahren folgendes umfasst:
a) das Hinzusetzen von NAPE-PLD oder Abh4 zu mindestens einem Aufnahmegefäß in einer Anordnung von Aufnahmegefäßen;
b) das Hinzusetzen eines Wirkstoffes zu mindestens einem Aufnahmegefäß in der Anordnung von Aufnahmegefäßen;
c) das Hinzusetzen eines Substrats zu mindestens einem Aufnahmegefäß in der Anordnung von Aufnahmegefäßen, wobei das Substrat eine Donoreinheit und eine Quenchereinheit aufweist, wobei die intramolekulare Nähe der Donoreinheit und der Quenchereinheit zu einem gequenchten detektierbaren Signal führt;
d) das Inkubieren des Wirkstoffes, des NAPE-PLD oder Abh4 und des Substrats in der Anordnung von Aufnahmegefäßen für eine vorbestimmte Zeitdauer;
e) das Messen der Menge an detektierbarem Signal von der Donoreinheit nach der Entfernung der Quenchereinheit von dem Substrat durch NAPE-PLD oder Abh4 in der Anordnung von Aufnahmegefäßen; und
f) das Vergleichen der Menge an detektierbarem Signal in einem ersten Aufnahmegefäß, welches den Wirkstoff, das Substrat und das NAPE-PLD oder Abh4 umfasst, mit der Menge an detektierbarem Signal in einem zweiten Aufnahmegefäß, welches das Substrat und das NAPE-PLD oder Abh4 umfasst,
wodurch der Wirkstoff als ein modifizierender Wirkstoff erkannt wird, wenn der Unterschied eine vorbestimmte Schwelle überschreitet,
wobei die Donoreinheit und die Quenchereinheit aus der Gruppe ausgewählt werden, die aus BODIPY/DNP, Rhodamin/DNP und Cy-Farbstoff/DNP besteht.

2. Verfahren gemäß Anspruch 1, wobei die Schritte (d) bis (f) durch die folgenden Schritte (d) bis (g) ersetzt werden:
d) das Messen der Menge an detektierbarem Signal von der Donoreinheit nach der Entfernung der Quenchereinheit von dem Substrat durch NAPE-PLD oder Abh4 in der Anordnung von Aufnahmegefäßen zu einem ersten Zeitpunkt;
e) das Inkubieren des Wirkstoffes, des NAPE-PLD oder Abh4 und des Substrats in der Anordnung von Aufnahmegefäßen für eine vorbestimmte Zeitdauer;
f) das Messen der Menge an detektierbarem Signal von der Donoreinheit nach der Entfernung der Quenchereinheit von dem Substrat durch NAPE-PLD oder Abh4 in der Anordnung von Aufnahmegefäßen zu einem zweiten Zeitpunkt; und
g) das Vergleichen der Menge an detektierbarem Signal zum ersten Zeitpunkt mit der Menge an detektierbarem Signal zum zweiten Zeitpunkt, wodurch der Wirkstoff als ein modifizierender Wirkstoff erkannt wird, wenn der Unterschied eine vorbestimmte Schwelle überschreitet.

3. Verfahren zur Erkennung eines Wirkstoffes, welcher die Aktivität von N-Acylphosphatidylethanolaminhydrolysierender Phospholipase D (NAPE-PLD) oder alpha/beta-Hydrolase-4 (Abh4) modifiziert, wobei das Verfahren folgendes umfasst:
a) das Hinzusetzen von NAPE-PLD oder Abh4 zu mindestens einem Aufnahmegefäß in der Anordnung von Aufnahmegefäßen;
b) das Hinzusetzen eines Wirkstoffes zu mindestens einem Aufnahmegefäß in der Anordnung von Aufnahmegefäßen;
c) das Hinzusetzen eines Substrats zu mindestens einem Aufnahmegefäß in der Anordnung von Aufnahmegefäßen, wobei das Substrat eine Donoreinheit und eine Akzeptoreinheit aufweist;
d) das Inkubieren des Wirkstoffes, des NAPE-PLD oder Abh4 und des Substrats für eine vorbestimmte Zeitdauer;
e) das Messen der Menge an detektierbarem Signal, wobei das gemessene detektierbare Signal aus der Gruppe ausgewählt wird, die aus der Donoreinheit, der Akzeptoreinheit, dem Verhältnis von Donoreinheit zur Akzeptoreinheit und dem Donor-Akzeptor-Einheiten-Komplex besteht; und
f) das Vergleichen der Menge an detektierbarem Signal in einem ersten Aufnahmegefäß, welches den Wirkstoff, das Substrat und das NAPE-PLD oder Abh4 enthält, mit der Menge an detektierbarem Signal in einem zweiten Aufnahmegefäß, welches das Substrat und das NAPE-PLD oder Abh4 enthält,
wodurch der Wirkstoff als ein modifizierender Wirkstoff erkannt wird, wenn der Unterschied eine vorbestimmte Schwelle überschreitet,
wobei die Donoreinheit und die Akzeptoreinheit aus der Gruppe ausgewählt werden, die aus Texas-Rot und Fluoreszein, BODIPY und Fluoreszein, Europium und Allophcocyamin (APC), Cyan-fluoreszierendem Protein/ gelb fluoreszierendem Protein und grün fluoreszierendem Protein/blau fluoreszierendem Protein besteht.

4. Verfahren gemäß Anspruch 3, wobei die Schritte (c) bis (f) durch die Schritte (c) bis (g) wie folgt ersetzt sind:
c) das Hinzusetzen eines Substrats zu mindestens einem Aufnahmegefäß in der Anordnung von Aufnahmegefäßen, wobei das Substrat eine Donoreinheit und eine Akzeptoreinheit aufweist;
d) das Messen der Menge an detektierbarem Signal zu einem ersten Zeitpunkt, wobei das gemessene detektierbare Signal aus der Gruppe ausgewählt wird, die aus der Donoreinheit, der Akzeptoreinheit, dem Verhältnis von Donoreinheit zur Akzeptoreinheit und dem Donor-Akzeptor-Einheiten-Komplex besteht; und
e) das Inkubieren des Wirkstoffes, des NAPE-PLD oder Abh4 und des Substrats für eine vorbestimmte Zeitdauer;
f) das Messen der Menge an detektierbarem Signal zu einem zweiten Zeitpunkt, wobei das gemessene detektierbare Signal aus der Gruppe gewählt wird, die aus der Donoreinheit, der Akzeptoreinheit, dem Verhältnis von Donoreinheit zur Akzeptoreinheit und dem Donor-Akzeptor-Einheiten-Komplex besteht; und
g) das Vergleichen der Menge an detektierbarem Signal zu dem ersten Zeitpunkt mit der Menge an detektierbarem Signal zu dem zweiten Zeitpunkt,
wodurch der Wirkstoff als ein modifizierender Wirkstoff erkannt wird, wenn der Unterschied eine vorbestimmte Schwelle überschreitet.

5. Verfahren gemäß Anspruch 3 oder 4, wobei das detektierbare Signal durch Fluoreszenz-Resonanz-Energietransfer gemessen wird.

6. Verfahren gemäß Anspruch 1, 2, 3 oder 4, weiterhin umfassend die Inkubation des Wirkstoffs und des NAPE-PLD oder Abh4 für eine vorbestimmte Zeitdauer vor dem Schritt c.

7. Verfahren gemäß Anspruch 1, 2, 3 oder 4, wobei die Schritte a, b und c in beliebiger Reihenfolge durchgeführt werden können oder zusammen durchgeführt werden können.

8. Verfahren gemäß Anspruch 1, 2, 3 oder 4, weiterhin umfassend mindestens eine Kontrolle.

9. Verfahren gemäß Anspruch 1, 2, 3 oder 4, weiterhin umfassend mindestens einen Inhibitor, Inducer oder Modulator.

10. Verfahren gemäß Anspruch 1, 2, 3 oder 4, wobei das Substrat PED6 ist

11. Verfahren gemäß Anspruch 10, wobei das PED6 in Ethylacetat gelöst ist.

12. Verfahren gemäß Anspruch 11, wobei das gelöste PED6 zu einem Puffer gegeben wird, welcher etwa 0,005 bis 0,1 % Triton X-100 umfasst.

13. Verfahren gemäß Anspruch 12, wobei der Puffer etwa 0,05 Triton X-100 umfasst.

14. Verfahren gemäß Anspruch 1, 2, 3 oder 4, wobei das detektierbare Signal aus der Gruppe ausgewählt wird, die aus einem Fluorochrom, Chromophor, Enzym, Linkereinheit, Biotin, Elektronendonoren, Elektronenakzeptoren, Farbstoffen, Metallen und Radionuklid besteht.

15. Verfahren gemäß Anspruch 1, 2, 3 oder 4, wobei das NAPE-PLD oder Abh4 in Schritt a bereitgestellt wird durch Kultivieren einer prokaryontischen oder eukaryontischen Zelle in dem Aufnahmegefäß, welche transient oder stabil transfiziert ist, um das NAPE-PLD oder Abh4 zu exprimieren.

16. Verfahren gemäß Anspruch 1, 2, 3 oder 4, wobei das NAPE-PLD oder Abh4 bereitgestellt wird nach der Isolation aus einer biologischen Probe oder von Zellen, welche transient oder stabil transfiziert sind, um NAPE-PLD oder Abh4 zu exprimieren.

17. Verfahren gemäß Anspruch 18, wobei die biologische Probe von einem Säuger ist, ausgewählt aus der Gruppe, die aus Mensch, Affe, Ratte, Maus, Kaninchen, und Meerschweinchen besteht.

18. Verfahren gemäß Anspruch 1, 2, 3 oder 4, wobei ein oder mehrere Schritt durch ein Roboter- oder Workstation-Gerät durchgeführt wird.

19. Assay-System zur Erkennung eines Wirkstoffes, welcher die Aktivität von NAPE-PLD oder Abh4 modifiziert, wobei das Assay-System umfasst:
a) eine Anordnung von Aufnahmegefäßen;
b) NAPE-PLD oder Abh4; und
c) ein Substrat, welches eine fluoreszierende Donoreinheit und eine Quenchereinheit umfasst, wobei die Donoreinheit zur Detektion in der Lage ist.

20. Assay-System zur Erkennung eines Wirkstoffes, welcher die Aktivität von NAPE-PLD oder Abh4 modifiziert, wobei das Assay-System umfasst:
a) eine Anordnung von Aufnahmegefäßen;
b) NAPE-PLD oder Abh4; und
c) ein Substrat, welches eine fluoreszierende Donoreinheit und eine Akzeptoreinheit umfasst, wobei die Donoreinheit oder die Akzeptoreinheit oder beide zur Detektion in der Lage ist bzw. sind.

## Revendications

1. Procédé d'identification d'un agent qui modifie l'activité de la phospholipase D hydrolysant la N-acylphosphatidyléthanolamine (NAPE-PLD) ou alpha-bêta-hydrolase-4 (Abh4), le procédé comprenant :
a) l'ajout de NAPE-PLD ou Abh4 à au moins un réceptacle dans une matrice de réceptacles ;
b) l'ajout d'un agent à au moins un réceptacle dans la matrice de réceptacles ;
c) l'ajout d'un substrat à au moins un réceptacle dans la matrice de réceptacles où le substrat comprend un fragment donneur et un fragment inactiveur où la proximité intramoléculaire du fragment donneur et du fragment inactiveur conduit à un signal détectable inactivé ;
d) l'incubation de l'agent, NAPE-PLD ou Abh4 et le substrat dans la matrice de réceptacles pendant une durée prédéterminée ;
e) la mesure de la quantité de signal détectable du fragment donneur après élimination du fragment inactiveur du substrat par NAPE-PLD ou Abh4 dans la matrice de réceptacles ; et
f) la comparaison de la quantité de signal détectable dans un premier réceptacle comprenant l'agent, le substrat et NAPE-PLD ou Abh4 à la quantité de signal détectable dans un deuxième réceptacle comprenant le substrat et NAPE-PLD ou Abh4
de manière à identifier ledit agent en tant qu'agent modificateur lorsque la différence dépasse un seuil prédéterminé
où le fragment donneur et le fragment inactiveur sont choisis dans le groupe constitué de BODIPY/DNP, Rhodamine/DNP et colorant Cy/DNP.

2. Procédé selon la revendication 1 dans lequel les étapes (d) à (f) sont remplacées par les étapes (d) à (g) suivantes
d) mesure de la quantité de signal détectable du fragment donneur après élimination du fragment inactiveur du substrat par NAPE-PLD ou Abh4 dans la matrice de réceptacles à un premier temps ;
e) incubation de l'agent, NAPE-PLD ou Abh4 et le substrat dans la matrice de réceptacles pendant un temps prédéterminé ;
f) mesure de la quantité de signal détectable du fragment donneur après élimination du fragment inactiveur du substrat par NAPE-PLD ou Abh4 dans la matrice de réceptacles à un deuxième temps ; et
g) comparaison de la quantité de signal détectable au premier temps à la quantité de signal détectable au deuxième temps, de manière à identifier ledit agent en tant qu'agent modificateur lorsque la différence dépasse un seuil prédéterminé.

3. Procédé d'identification d'un agent qui modifie l'activité de phospholipase D hydrolysant la N-acylphosphatidyléthanolamine (NAPE-PLD) ou alpha-bêta-hydrolase-4 (Abh4), le procédé comprenant :
a) l'ajout de NAPE-PLD ou Abh4 à au moins un réceptacle dans une matrice de réceptacles ;
b) l'ajout d'un agent à au moins un réceptacle dans la matrice de réceptacles ;
c) l'ajout d'un substrat à au moins un réceptacle dans la matrice de réceptacles où le substrat comprend un fragment donneur et un fragment accepteur ;
d) l'incubation de l'agent, NAPE-PLD ou Abh4 et le substrat pendant un temps prédéterminé ;
e) la mesure de la quantité de signal détectable où le signal détectable mesuré est choisi dans le groupe constitué du fragment donneur, du fragment accepteur, du rapport de fragment donneur au fragment accepteur et du complexe fragment donneur-fragment accepteur ; et
f) la comparaison de la quantité de signal détectable dans un premier réceptacle comprenant l'agent, le substrat et NAPE-PLD ou Abh4 à la quantité de signal détectable dans un deuxième réceptacle comprenant le substrat et NAPE-PLD ou Abh4
de manière à identifier ledit agent en tant qu'agent modificateur lorsque la différence dépasse un seuil prédéterminé
où le fragment donneur et le fragment accepteur sont choisis dans le groupe constitué du rouge Texas et la fluorescéine, BODIPY et la fluorescéine, l'europium et l'allophcocyamine (APC), la protéine fluorescente cyan/protéine fluorescente jaune, et la protéine fluorescente verte/protéine fluorescente bleue.

4. Procédé selon la revendication 3, dans lequel les étapes (c) à (f) sont remplacées par les étapes (c) à (g) comme suit :
c) l'ajout d'un substrat à au moins un réceptacle dans la matrice de réceptacles où le substrat comprend un fragment donneur et un fragment accepteur ;
d) la mesure de la quantité de signal détectable à un premier temps où le signal détectable mesuré est choisi dans le groupe constitué du fragment donneur, du fragment accepteur, du rapport du fragment donneur au fragment accepteur et du complexe fragment donneur-fragment accepteur ;
e) l'incubation de l'agent, NAPE-PLD ou Abh4 et le substrat pendant un temps prédéterminé ;
f) la mesure de la quantité de signal détectable à un deuxième temps où le signal détectable mesuré est choisi dans le groupe constitué du fragment donneur, du fragment accepteur, du rapport du fragment donneur au fragment accepteur et du complexe fragment donneur-fragment accepteur ; et
g) la comparaison de la quantité de signal détectable au premier temps à la quantité de signal détectable au deuxième temps
de manière à identifier ledit agent en tant qu'agent modificateur lorsque la différence dépasse un seuil prédéterminé.

5. Procédé de la revendication 3 ou 4 dans lequel le signal détectable est mesuré par transfert d'énergie de fluorescence par résonance.

6. Procédé de la revendication 1, 2, 3, ou 4 comprenant en outre l'incubation de l'agent et NAPE-PLD ou Abh4 pendant un temps prédéterminé avant l'étape c.

7. Procédé de la revendication 1, 2, 3 ou 4 dans lequel les étapes a, b et c peuvent être conduites dans un ordre quelconque ou peuvent être conduites conjointement.

8. Procédé de la revendication 1, 2, 3 ou 4 comprenant en outre au moins un témoin.

9. Procédé de la revendication 1, 2, 3 ou 4 comprenant en outre au moins un inhibiteur, inducteur ou modulateur.

10. Procédé de la revendication 1, 2, 3 ou 4 dans lequel le substrat est PED6.

11. Procédé de la revendication 10 dans lequel PED6 est dissous dans de l'acétate d'éthyle.

12. Procédé de la revendication 11 dans lequel PED6 dissous est ajouté à un tampon comprenant environ 0,005 à 0,1 % de Triton X-100.

13. Procédé de 12 dans lequel le tampon comprend environ 0,05 % de Triton X-100.

14. Procédé de la revendication 1, 2, 3 ou 4 dans lequel le signal détectable est choisi dans le groupe constitué d'un fluorochrome, un chromophore, une enzyme, un fragment lieur, la biotine, des donneurs d'électron, des accepteurs d'électron, des colorants, des métaux, et de la radionucléide.

15. Procédé de la revendication 1, 2, 3 ou 4 dans lequel NAPE-PLD ou Abh4 dans l'étape a est produit par culture dans le réceptacle d'une cellule procaryote ou eucaryote qui est transfectée de façon transitoire ou stable pour exprimer NAPE-PLD ou Abh4.

16. Procédé de la revendication 1, 2, 3 ou 4 dans lequel NAPE-PLD ou Abh4 est produit après isolement à partir d'un échantillon biologique ou de cellules transfectées de façon transitoire ou stable pour exprimer NAPE-PLD ou Abh4.

17. Procédé de la revendication 18 dans lequel l'échantillon biologique provient d'un mammifère choisi dans le groupe constitué d'un humain, un singe, un rat, une souris, un lapin, et un cobaye.

18. Procédé de la revendication 1, 2, 3 ou 4 dans lequel une ou plusieurs étapes sont conduites par un dispositif robotique ou une station de travail.

19. Système d'essai pour identifier un agent qui modifie l'activité de NAPE-PLD ou Abh4, le système d'essai comprenant :
a) une matrice de réceptacles ;
b) NAPE-PLD ou Abh4 ; et
c) un substrat comprenant un fragment donneur fluorescent et un fragment inactiveur où le fragment donneur est capable de détection.

20. Système d'essai pour identifier un agent qui modifie l'activité de NAPE-PLD ou Abh4, le système d'essai comprenant :
a) une matrice de réceptacles ;
b) NAPE-PLD ou Abh4 ;
c) un substrat comprenant un fragment donneur fluorescent et un fragment accepteur où le fragment donneur ou le fragment accepteur ou les deux sont capables de détection.
